(19) Europäisches Patentamt
European Patent Office
Office européen des brevets

(11) EP 0 966 464 B1

(12) EUROPEAN PATENT SPECIFICATION

(45) Date of publication and mention of the grant of the patent:
**28.05.2003 Bulletin 2003/22**

(21) Application number: **98913585.0**

(22) Date of filing: **24.02.1998**

(51) Int Cl.[7]: **C07D 403/10**, A61K 31/505, C07D 405/14, C07D 403/14, C07D 401/14

(86) International application number:
**PCT/EP98/01051**

(87) International publication number:
**WO 98/039328 (11.09.1998 Gazette 1998/36)**

(54) **SUBSTITUTED 2,4-DIAMINOPYRIMIDINES**

SUBSTITUIERTE 2,4-DIAMINOPYRIMIDINE

2,4,-DIAMINOPYRIMIDINES SUBSTITUES

(84) Designated Contracting States:
**AT BE CH DE DK ES FI FR GB GR IE IT LI LU NL PT SE**

(30) Priority: **03.03.1997 EP 97103436**

(43) Date of publication of application:
**29.12.1999 Bulletin 1999/52**

(73) Proprietor: **Basilea Pharmaceutica AG**
**4102 Binningen (CH)**

(72) Inventors:
- **GUERRY, Philippe**
  **CH-4102 Binningen (CH)**
- **HUBSCHWERLEN, Christian**
  **F-68200 Durmenach (FR)**
- **JOLIDON, Synèse**
  **CH-4223 Blauen (CH)**
- **SPECKLIN, Jean-Luc**
  **F-68680 Kembs-Schaferhof (FR)**
- **WYSS, Pierre-Charles**
  **CH-4132 Muttenz (CH)**

(74) Representative: **Schager, Frank, Dr.**
**F. Hoffmann-La Roche AG,**
**Patent Department (CLP),**
**124 Grenzacherstrasse**
**4070 Basel (CH)**

(56) References cited:
**WO-A-96/16046** **WO-A-97/43277**

- **B.ROTH ET AL: "2,4-Diamino-5-benzylpyrimidines as Antibacterial agents" JOURNAL OF MEDICINAL CHEMISTRY, Bd. 32, Nr. 8, 1989, Seiten 1949-1958, XP002068896**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 966 464 B1

## Description

The present invention is concerned with substituted 2,4-diamino-pyrimidines of the general formula

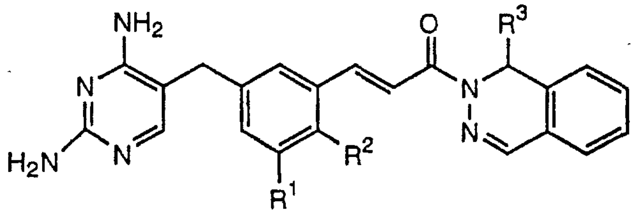

I

wherein

| | |
|---|---|
| $R^1$ | signifies $C_{1-4}$-alkoxy or $C_{1-4}$-alkoxy substituted with amino, dialkylamino, morpholino, piperidino; piperazino, hydroxy, halide, nitrile, thiocyanato, sulfato, methylsulfanyl, oxo, carboxy, carbamino, carbalkoxy groups, alkoxy, morpholinoalkoxy or piperidinoalkoxy; |
| $R^2$ | signifies hydroxy, $C_{1-4}$-alkoxy or $C_{1-4}$-alkoxy substituted with amino, dialkylamino, morpholino, piperidino, piperazino, hydroxy, halide, nitrile, thiocyanato, sulfato, methylsulfanyl, oxo, carboxy, carbamino, carbalkoxy groups, alkoxy, morpholinoalkoxy or piperidinoalkoxy; |
| $R^3$ | signifies hydrogen, cyano, $C_{1-6}$-alkyl, substituted $C_{1-6}$-alkyl, up to $C_6$-alkenyl, substituted up to $C_6$-alkenyl, $C_{3-6}$-cycloalkyl, substituted $C_{3-6}$-cycloalkyl, bicyclyl, aryl, substituted aryl, heterocyclyl, substituted heterocyclyl, heteroaryl, substituted heteroaryl, aralkyl, substituted aralkyl, aryl-Q-alkyl, substituted aryl-Q-alkyl, heterocyclylalkyl, substituted heterocyclylalkyl or a group of the formula-$CR^4R^{4'}COR^5$,<br>wherein substituted $C_{1-6}$-alkyl or substituted $C_{3-6}$-cycloalkyl are substituted with amino, dialkylamino, morpholino, piperidino, piperazino, hydroxy, halide, nitrile, thiocyanato, sulfato, methylsulfanyl, oxo, carboxy, carbamino, carbalkoxy groups, alkoxy, morpholinoalkoxy or piperidinoalkoxy, and<br>wherein substituted up to $C_6$-alkenyl is subsituted with cyano or acryloyl, and<br>wherein heterocyclyl signifies 4 to 6 membered rings with 1 to 3 N, O and/or S atoms, and<br>wherein substituted aryl, substituted heterocyclyl or substituted heteroaryl are substituted with phenyl, $C_{1-6}$-alkyl, substituted $C_{1-6}$-alkyl, $C_{3-6}$ cycloalkyl, hydroxy, cyano, thiocyanato, amino, hydroxyalkyl (optionally esterified with amino acids or sulphuric acid), halogen, $C_{1-4}$-alkoxy, $C_{1-4}$- alkoxycarbonyl, di($C_{1-6}$-alkyl)amino, carbamoyl, mono- or di-$C_{1-6}$-alkylcarbamoyl, $C_{1-6}$-alkylsulphanoyl, $C_{1-6}$-alkylsulphonyl, sulphamoyl, N-mono- or di-$C_{1-6}$-alkylsulphamoyl, heterocyclyl, or heterocyclyl -$C_{1-6}$-alkyl, and<br>wherein heteroaryl signifies 5- or 6- membered, mono- or poly-nuclear heteroaromatic groups with preferably 5-13 carbon atoms and 1-4 hetero atoms; |
| Q | signifies -SO- or $SO_2$; |
| $R^4$, $R^{4'}$ | each independently signify hydrogen, alkyl, aryl, substituted aryl, heterocyclyl or substituted heterocyclyl, wherein heterocyclyl, substituted aryl and substituted heterocylyl are as defined for $R^3$; |
| $R^5$ | signifies hydrogen, alkyl, alkoxy, aryl, substituted aryl, heterocyclyl or substituted heterocyclyl, wherein heterocyclyl, substituted aryl, and substituted heterocylyl are as defined for $R^3$; |
| $R^4$ and $R^5$ | together can form a group $-(CH_2)_n-$, and |
| n | is a whole number of 2-5, |

as well as pharmaceutically usable salts thereof.

The compounds of formula I are novel and possess valuable antibiotic properties. They can be used for the control or prevention of infectious diseases. In particular, they exhibit a pronounced antibacterial activity even against multiresistant, gram-positive strains and against resistant pneumococci and opportunistic pathogens such e.g. Pneumocystis carinii. The compounds of formula I can also be administered in combination with known antibacterially-active substances and then exhibit synergistic effects. Typical combination partners are e.g. sulphonamides, with which the compounds of formula I or their salts can be admixed in various ratios.

Objects of the present invention are compounds of formula I and their pharmaceutically acceptable salts per se and for use as therapeutically active substances, medicaments based on these substances, optionally in combination with sulphonamides, and their production; the use of these substances as medicaments and for the production of antibacterially-active medicaments; as well as the manufacture of the compounds of formula I and their pharmaceutically acceptable salts and intermediates for their manufacture.

The terms "$C_1$-$C_6$ alkyl" and "$C_1$-$C_4$-alkoxy" are chain or branched alkyl and, respectively, alkoxy groups which are optionally substituted by one or more substituents, such as, for example, methyl, ethyl, n-propyl, isopropyl, n-butyl, isobutyl, tert.butyl, n-pentyl and n-hexyl; and, respectively, methoxy, ethoxy, n-propoxy, isopropoxy, n-butyloxy, isobutyloxy, and tert.butoxy. Suitable substituents for these alkyl and alkoxy groups are functional groups chosen from amino, dialkylamino, morpholino, piperidino, piperazino, hydroxy, halide, nitrile, thiocyanato, sulfato, methylsulfanyl, oxo, carboxy, carbamino or carbalkoxy groups or substituents such as alkoxy, morpholinoalkoxy, piperidinoalkoxy. Methoxymethyl, hydroxymethyl, hydroxybutyl, dihydroxybutyl, 2-oxo-propyl, 3-propionaldehyde, perfluorohexyl and the like are examples of such substituted alkyl and alkoxy groups.

The term "alkenyl" is unsaturated hydrocarbon residues with up to 6 carbon atoms containing a double bond such as vinyl, allyl, butenyl and the like. The alkenyl chain is obtionally substituted by one or more substituents, such as, for example, cyano and acryloyl.

The term "cycloalkyl" is cyclic alkyl groups with preferably 3-6 carbon atoms, which can be substituted with functional groups and substituents as mentioned under alkyl and alkoxy.

Examples for bicyclyl groups are adamantyl or bicyclo[2.2.1]hept-2endo-and/or 2exo-yl.

The term "aryl" denotes 6-membered mono- or poly-nuclear aromatic groups with preferably 6-14 carbon atoms. Examples of such groups are phenyl, naphthyl, anthryl and phenanthryl, which can be substituted by one or more substituents. Substituents for ether mentioned aryl groups are e.g. phenyl; lower-alkyl (e.g. methyl); substituted lower-alkyl (e.g. trifluoromethyl, pentafluoro ethyl); $C_{3-6}$ cycloalkyl (e.g. cyclopropyl); hydroxy; cyano; thiocyanato; amino; hydroxyalkyl, optionally esterified with amino acids or sulphuric acid (such as, for example, 2-amino-propionic acid ester or 2-amino-5-guanidino-pentanoic acid ester); halogen (e.g. chlorine); lower-alkoxy (e.g. methoxy, n-butoxy); lower alkoxycarbonyl (e.g. methoxycarbonyl); di(lower alkyl)amino (e.g. dimethylamino, diethylamino); carbamoyl, mono- or di-lower-alkylcarbamoyl; lower-alkylsulphanoyl, (e.g. methylsulphanyl); lower-alkylsulphonyl, (e.g. methanesulphonyl); sulphamoyl, N-mono- or di-lower alkylsulphamoyl; heterocyclyl, or with heterocyclyl-lower-alkyl.

The term "heterocyclyl" is in the scope of the present invention 4 to 6 membered rings with 1 to 3 N, O and/or S atoms such as, for example, cyclic lactones, cyclic lactames, ketals (such as, for example, 2-dimethyl-1, 3-dioxolan-yl), acetals (e.g. 1,3, dioxolan-2-yl or 1,3,-dioxan-2-yl); examples of such rings are morpholin-4-yl, 4-methyl-piperazin-1-yl, imidazol-1-yl, thiazolyl and [1,2,4] triazol-1-yl, dithianyl, tetrahydropyranyl; the heterocyclic rings can be substituted with substituents described above for the aryl groups. Such substituents are $C_1$-$C_6$ alkyl, $C_1$-$C_4$-alkoxy, hydroxy, amino, hydroxyalkyl, aminoalkyl or oxo. Pyrrolidinone, methylpyrrolidinone and the like are examples of preferred substituted heterocyclic rings.

The term "heterocyclyl-lower alkyl" is in the scope of the present invention heterocyclic rings which are linked via an alkyl residue. Preferred heterocyclyl-lower-alkyl units are e.g morpholin-4-ylmethyl, 4-methyl-piperazin-1-ylmethyl, imidazol-1-ylmethyl and [1,2,4]triazol-1-ylmethyl,dioxolan-4yl-ethyl, prrolidinylamethyl, pyperidinylmethyl and the like.

The term "heteroaryl" denotes in the scope of the present invention 5- or 6- membered, mono- or poly-nuclear heteroaromatic groups with preferably 5-13 carbon atoms and 1-4 hetero atoms, preferably nitrogen, oxygen and/or sulphur atoms. Furyl, pyranyl, thienyl, pyrrolyl, pyrazolyl, imidazolyl, triazolyl, tetrazolyl, oxazolyl, oxadiazolyl, isoxazolyl, thiazolyl, isothiazolyl, pyridyl, pyridazinyl, pyrimidinyl, pyrazinyl, triazinyl are examples. These groups can also be linked with a fused ring, preferably a phenyl ring. Examples of such linked rings are, for example, benzopyranyl, benzofuranyl, indolyl and quinolinyl. The heteroaryl groups can be substituted, for example with substituents as described above for the aryl groups.

In formula I the group $R^1$ is preferably methoxy; $R^2$ is preferably hydroxy, lower alkoxy such as e.g. methoxy or ethoxy; or lower alkoxy substituted by lower alkoxy such as e.g. methoxymethoxy, lower alkoxy substituted by heterocyclyl such as e.g. morpholin-4-yl-ethoxy or lower alkoxy substituted by lower alkoxycarbonyl-lower alkyl, preferably methoxycarbonylmethyl.

Preferred lower alkyl groups $R^3$ are the following groups:

methyl (Ex. 1.32, 1.33, 2.11); ethyl (Ex. 2.13); propyl (Ex. 1.30, 1.31, 2.15, 2.16); 3-methyl-butyl (Ex. 2.17); tert. butyl (Ex. 1.23).

Preferred substituents for the lower alkyl residue $R^3$ are the following substituents:

hydroxy such as e.g. hydroxypropyl (Ex. 2.18), hydroxybutyl (Ex. 2.20), 3,4-dihydroxybutyl (Ex. 16);
methylsulfanyl such as e.g. methylsulfanylmethyl (Ex.1.17);
fluor such as e.g. tridecafluorhexyl (Ex. 2.3);
carbamoyloxy such as e.g. carbamoyloxy- butyl (Ex. 2.21), carbamoyloxy-propyl (Ex. 2.22);
thiocyanato such as e.g. thiocyanatobutyl (Ex. 11);
-$SO_4H$ such as e.g. sulfatobutyl (Ex.10.2) or heterocyclyl such as e.g. [1.3]dioxolan-2yl-ethyl (Ex. 2.23), [1.3]dioxolan-4yl-ethyl (Ex. 4).

Preferred substituents for the alkenyl-residue $R^3$ are the following substituents:

cyano such as e.g. cyanobutenyl (Ex.13.1);
acryloyl such as e.g. acryloylbutenyl (Ex.13.2).

Preferred lower cycloalkyl groups $R^3$ are the following groups:

cyclopropyl (Ex 1.46 1.52, 1.53), cyclobutyl (Ex. 1.56), cyclopentyl (Ex.1.57), cyclohexyl (Ex.1.60).

A preferred substituent for the cycloalkyl residue $R^3$ is the oxo-group such as e.g. cyclopentanon (Ex.1.9), cyclohexanon (Ex.1.10).

Preferred heterocyclyl- or substituted heterocyclyl-residues R3 are:

dithian-2-yl (Ex. 1.49) or tetrahydropyran-2-on 1 (Ex. 1.14).

Examples for the group "aryl -Q-alkyl" are: phenylsulfonylmethyl (Ex. 1.69) or Phenylsulfinylmethyl (Ex. 2.5).

Examples for the group "$CR^4R^{4'}COR^5$" are: methylcarbonylmethyl (Ex.1.11), methoxycarbonyl(dimethyl) methano $CH_3$-CO-C$(CH_3)_2$- (Ex. 1.12), hydroxycarbonyldimethylmethano (-C$(CH_3)_2$-CO-OH (Ex. 7), phenylcarbonyl-methyl (Ex.1.13), phenylcarbonyl(methyl)methyl (Ex.1.15), furanylcarbonyl-methyl (Ex.1.16) or morpholinylcarbonyloxybutyl (Ex. 2.19).

Preferred aryl groups $R^3$ are phenyl or biphenyl, most preferred phenyl.

The phenyl residue is optionally mono, di-or tri substituted by the following groups:

lower alkyl such as e.g. methyl (Ex. 1.19, 1.22, 1.25, 1.40, 1.41), ethyl (Ex. 1.20), butyl (Ex. 1.3), tert. butyl (Ex. 1.18);
substituted lower alkyl such as e.g. hydroxymethyl (Ex. 2.6, 2.8, 2.9, 2.12, 3.3, 8.3), hydroxy-ethyl (Ex. 1.68, 2.12), methoxymethyl ( Ex. 1.71), trifluormethyl (Ex. 1.58), dimethylaminomethyl (Ex. 1.7), diisopropylamino-methyl (Ex. 1.72), 2-aminopropionylmethyl (Ex. 9.1), carbamoyloxymethyl (Ex. 2.14);
lower alkoxy, preferably methoxy (Ex. 1.62, 32.6);
halogen, preferably fluoro (Ex. 2.1, 2.2), chloro (Ex. 1.73);
methylsulfanyl (Ex. 1.47, 1.54, 1.55);
dimethylamino (Ex. 1.38);
dimethylamino sulfonyl ( Ex. 1.6, 1.70);
cyano (Ex. 1.64);
hydroxy (Ex. 2.7, 2.10);
lower alkoxy such as e.g. methoxy (Ex. 1.61, 32.3);
substituted lower alkoxy such as e.g. hydroxyethoxy ( Ex. 32.11), trifluormethoxy (Ex. 1.21), 1-ethoxy-ethoxy (Ex. 1.35), 2-ethoxy-ethoxy (Ex.1.39);
lower alkoxy-carbonyl such as e.g. tert. butoxy carbonyl (Ex. 1.65); heteroaryl such as e.g. prrrol-1-yl (Ex. 1.42), furan-2-yl (Ex. 1.28, 1.29, 1.4, 3.2, 8.2), thiazol-2yl (Ex. 1.5);
heterocyclyl-lower alkyl such as e.g. 4-methyl-piperazin-1-ylmethyl (Ex. 1.75), 4-morpholin-4-yl-methyl (Ex.1.76).

Preferred heteroaryl groups $R^3$ are pyridyl (Ex. 1.51, 1.2, 32.8) pyrimidinyl, thiophen-2-yl (Ex. 1.66), 5,6-dihydro-4H-pyran-2-yl (Ex. 1.24), [1,3]dioxolo[4,5-b]pyridin-6-yl (Ex. 32.13).

The heteroaryl groups $R^3$ is optionally mono, di-or tri substituted by the following groups:

lower alkyl such as e.g. methyl (Ex. 1.37, 1.43, 1.44, 1.48, 1. 67, 1.8, 32.1, 32.2, 32.432.5), ethyl (Ex. 34.1);
substituted lower alkyl such as e.g. hydroxymethyl (Ex. 32.7), hydroxy-1-methyl-ethyl (Ex. 1.78);
halogen, preferably chloro (Ex. 1.59), bromo (Ex. 32.9);
lower alkoxy such as e.g. methoxy (Ex. 1.74, 32.10, 32.12);
substituted lower alkoxy such as e.g. methoxy-ethoxy (Ex. 32.15), methoxy-ethoxy-ethoxy ( Ex. 32.16), hydroxyethoxy (Ex. 32.14), hydroxypropoxy (Ex. 32.17), 2-morpholin-4-yl-ethoxy (Ex. 32.19), dimethylamino-ethoxy (Ex. 32.20); benzyloxy (Ex. 32.18);
dimethylamino (Ex. 1.50);
amino-carbonyl (Ex. 34);
tert. butyl amino carbonyl (Ex.1.80);
heterocyclyl such as e.g. morpholin-4-yl (Ex. 1.79).

Preferred compounds of formula I are those in which $R^1$ and $R^2$ signify lower-alkoxy, especially methoxy; $R^3$ signifies phenyl, or substituted phenyl or furyl, or lower-alkoxy. The compounds of formula I in which $R^3$ is different from hydrogen can be present in racemic form or as the R- or S-enantiomer. Examples of preferred compounds of formula I are:

A: (E)-(RS)-3-[5-(2,4-Diamino-pyrimidin-5-ylmethyl)-2,3-dimethoxyphenyl]-1-(1-phenyl-1H-phthalazin-2-yl)-propenone; (Example 1.1.)

B: (E)-(RS)-3-[5-(2,4-Diamino-pyrimidin-5-ylmethyl)-2,3-dimethoxyphenyl]-1-(1-furan-2-yl-1H-phthalazin-2-yl)-propenone; Example 1.4.)

C: (E)-(RS)-3-[5-(2,4-Diamino-pyrimidin-5-ylmethyl)-2,3-dimethoxyphenyl]-1-[1-(4-hydroxymethyl-phenyl)-1H-phthalazin-2-yl]-propenone (Example 2.6.)

D: (E)-(RS)-3-[5-(2,4-Diamino-pyrimidin-5-ylmethyl)-2,3-dimethoxyphenyl]-1-[1-(4-hydroxy-phenyl)-1H-phthalazin-2-yl]-propenone (Example 2.7.)

E: (E)-(RS)-3-[5-(2,4-Diamino-pyrimidin-5-ylmethyl)-2,3-dimethoxyphenyl]-1-(1-ethyl-1H-phthalazin-2-yl)-propenone (Example 2.13.)

F: (E)-(RS)-3-[5-(2,4-Diamino-pyrimidin-5-ylmethyl)-2,3-dimethoxyphenyl]-1-[1-(4-hydroxy-butyl)-1H-phthalazin-2-yl]-propenone (Example 2.20.)

G: (E)-(RS)-3-{5-(2,4-Diamino-pyrimidin-5-ylmethyl)-2,3-dimethoxyphenyl}-1-(1-propyl-1H-phthalazin-2-yl)-propenone; (Example 2.15) as well as pharmaceutically usable salts of these compounds.

Furthermore, compounds of formula I are preferred wherein $R^3$ is pyridin-yl or pyrimidinyl optionally substituted by lower alkyl such as e.g

H: (E)-(R)-3-[5-(2,4-Diamino-pyrimidin-5-ylmethyl)-2,3-dimethoxy-phenyl]-1-[1-(6-methyl-pyridin-3-yl)-1H-phthalazin-2-yl]-propenone (Example 1.43);

I: (E)-(RS)-3-[5-(2,4-Diamino-pyrimidin-5-ylmethyl)-2,3-dimethoxyphenyl)-1-[1-(6-methyl-pyridin-3-yl)-1H-phthalazin-2-yl]-propenone (Example 1.48),

J: (E)-(RS)-3-[5-(2,4-Diamino-pyrimidin-5-ylmethyl)-2,3-dimethoxyphenyl]-1-[1-(6-ethyl-pyridin-3-yl)-1H-phthalazin-2-yl]-propenone (Example 34.1),

K: (E)-(RS)-3-[5-(2,4-Diamino-pyrimidin-5-ylmethyl)-2,3-dimethoxyphenyl]-1-[1-(2-methyl-pyrimidin-5-yl)-1H-phthalazin-2-yl]-propenone (Example 32.5).

The compounds of formula I form pharmaceutically acceptable acid addition salts with organic and inorganic acids. Examples of acid addition salts of compounds of formula I are salts with mineral acids, for example hydrohalic acids, such as hydrochloric acid, hydrogen bromide and hydrogen iodide, sulphuric acid, nitric acid, phosphoric acid and the like, salts with organic sulphonic acids, for example with alkyl- and arylsulphonic acids such as methanesulphonic, p-toluenesulphonic, benzen-sulphonic acid and the like, as well as salts with organic carboxylic acids, for example with acetic acid, tartaric acid, maleic acid, citric acid, benzoic acid, salicylic acid, ascorbic acid and the like.
The compounds of formula I and their pharmaceutically acceptable salts can be manufactured in accordance with the invention by

(a) reacting a compound of the general formula

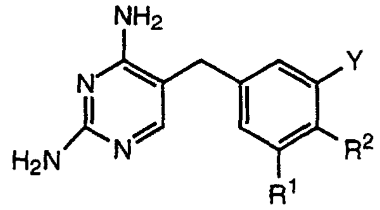

II

wherein $R^1$ and $R^2$ have the above significance and Y signifies a leaving group, with a compound of the general formula

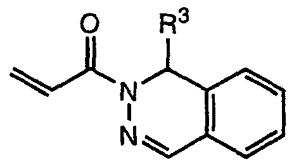

III

wherein $R^3$ is as defined above, or

(b) if desired, functionally modifying reactive groups present in the reaction product, or

c) converting a compound of formula I into a pharmaceutically acceptable salt.

In order to manufacture end products of formula I in accordance with process variant a) of the process in accordance with the invention, a so-called "Heck reaction" is carried out by e.g. reacting a starting compound of formula II in which the leaving group Y represents, for example, bromine, iodine, methanesulphonyloxy, trifluoromethanesulphonyloxy, benzenesulphonyloxy or p-toluenesulphonyloxy with a compound of general formula III. Preferably, an inert organic solvent, e.g. dioxan, tetrahydrofuran, N,N-dimethylacetamide or N,N-dimethylformamide, is used. The reaction is preferably effected in the presence of a base such as alkali metal carbonate or hydrogen carbonate, e.g. potassium carbonate or sodium hydrogen carbonate, and/or a tertiary amine, e.g. in a tri(lower alkyl)amine such as triethylamine, tri-n-butylamine or N-ethylpiperidine, and together with a catalyst, preferably a palladium complex, such as palladium (II) acetate, bis(triphenyl-phosphine) palladium(II) dichloride, bis(triphenyl-phosphine)palladium(II) diacetate, tetrakistriphenylphosphine palladium, or copper(I) iodide and triphenylphosphine or tri-o-tolylphosphine, optionally with the addition of a phase transfer catalyst such as a tetraalkylammonium salt, e.g. tetrabutylammonium bromide. The temperature of the "Heck reaction" preferably lies in the region between about 40°C and the boiling point of the reaction mixture.

If desired, reactive groups present in the reaction product can be functionally modified in accordance with variant b) of the process in accordance with the invention. For example, in reaction products of formula I groups $R^3$ which contain alcohol functions can be transformed into an ester.

The manufacture of the salts of the compounds of formula I in accordance with variant c) can be effected in a manner known per se, e.g. by reacting a compound of formula I with an organic or inorganic acid, conveniently in a solvent such as acetone, ethanol, methanol or water.

The compounds of formula III can be obtained in accordance with the invention by

a) reacting a compound of the general formula

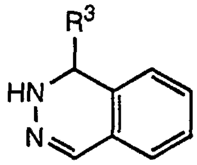

IV

in which $R^3$ has the above significance with a reactive acrylic acid derivative.

Examples of reactive acrylic acid derivatives are the acid halides, especially the acid chloride, reactive amides, such as, for example, the imidazolide, and mixed anhydrides. The acylation in accordance with the invention can be carried out in an inert solvent, e.g. a hydrocarbon such as benzene or toluene, a chlorinated hydrocarbon such as chloroform or methylene chloride, or an ether such as dioxan or tetrahydrofuran, in the presence of a base, e. g. an amine such as pyridine or triethylamine (which can simultaneously serve as the solvent). The reaction temperature is not critical. The reaction is conveniently performed at temperatures between 0°C and 50°C, especially at 0°C to 30°C;
or, especially in the case of compounds of formula III in which $R^3$ signifies a group of the formula $-CR^4R^{4'}COR^5$, by

b) reacting a phthalazine in accordance with the following Reaction Scheme with a silyl enol ether of formula V of an enamine of formula VI in the presence of a reactive acrylic acid derivative such as, for example, the acid halide:

Reaction A

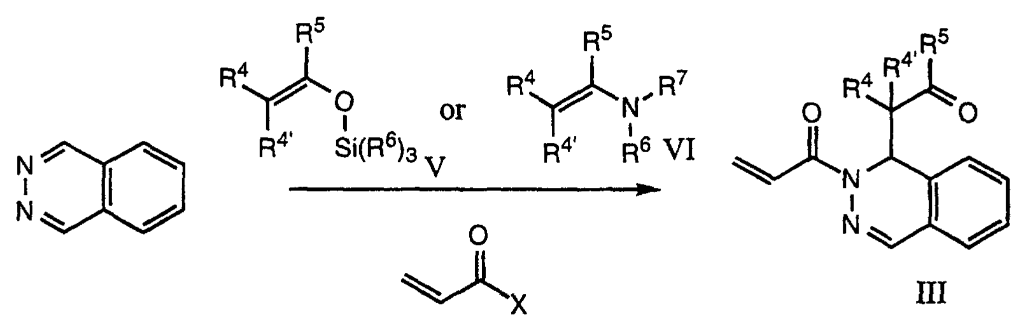


V

VI

III

wherein

| | |
|---|---|
| $R^4$, $R^{4'}$ | each independently signify hydrogen, alkyl, aryl or heterocyclyl, |
| $R^5$, | signifies hydrogen, alkyl, alkoxy, aryl or hetro-cyclyl, or |
| $R^4$ and $R^5$ | together can form a group $-(CH_2)_n-$, |
| n | is a whole number of 2 to 5, |
| $R^6$-$R^7$ | signify lower alkyl, |
| and $R^6$ and $R^7$ | in the case of enamines of formula VI can also together form a ring, |
| X | signifies a halogen atom such as e.g. chlorine or bromine. |

This reaction is preferably effected in inert solvents such as ether or in chlorinated hydrocarbons at temperatures of -20°C to +20°C with subsequent hydrolysis of the reaction product at 0 to 50°C.
The compounds of formula IV can be prepared in accordance with the following Reaction Scheme

Reaction B

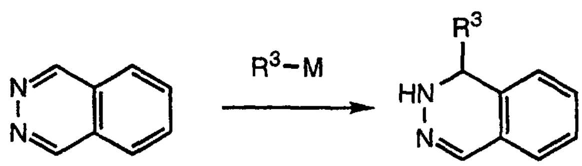


IV

wherein

$R^3$ has the above significance and

M signifies Li, Na, MgBr, MgCl or MgI.

This reaction is preferably effected in a temperature range of -80°C to 20°C. An open-chain or cyclic ether such as diethyl ether or tetrahydrofuran is preferably used as the solvent.

As mentioned earlier, the compounds of formula I and their pharmaceutically acceptable salts possess valuable antibacterial properties. They are active against a large number of pathogenic microorganisms such as e.g. Staphylococcus aureus, Pneumocystis carinii, Strepococcus pneumoniae etc. by virtue of their activity in inhibiting bacterial dihydrofolate reductase (DHFR). The inhibition of this enzyme was taken as a measurement of the antibacterial activity. It is determined by the method of D.P. Baccanari and S.S. Joyner (Biochemistry 20, 1710 (1981)); see also P.G. Hartman et al., FEBS Letters 242, 157 (1988).

The $IC_{50}$-values (concentration at which the enzyme is inhibited by 50%) are determined by means of a graph.

The following Table contains inhibitory concentrations determined for representative members of the class of compound defined by formula I and determined in the above test. The $IC_{50}$ values (μM) against the purified DHFR of the reference strain S. aureus ATCC 25923, as well as against the purified DHFR of the multiresistant strain S. aureus 157/4696 are given. The third column shows $IC_{50}$ values (μM) against the purified DHFR of the multiresistant strain Streptococcus pneumoniae 1/1. The inhibition constants of trimethoprim are also given as a comparison.

| Example | S.aureus ATCC25923 | S.aureus 157/4696 | S.pneumoniae 1/1 |
|---|---|---|---|
| Trimethoprim | 0.034 | 16.000 | 3.000 |
| A | 0.002 | 0.002 | 0.012 |
| B | 0.002 | 0.008 | 0.022 |
| C | 0.001 | 0.001 | 0.002 |
| D | 0.002 | 0.003 | 0.009 |
| E | 0.002 | 0.012 | 0.018 |
| F | 0.001 | 0.003 | 0.009 |
| G | 0.002 | 0.006 | 0.005 |
| H | 0.0003 | 0.0004 | 0.0012 |
| I | 0.0004 | 0.0004 | 0.0004 |
| J | 0.0003 | 0.0002 | 0.0015 |
| K | 0.0006 | 0.0006 | 0.0025 |

The products in accordance with the invention can be used as medicaments, e.g. in the form of pharmaceutical preparations for enteral or parenteral administration. For example, the products in accordance with the invention can be administered perorally, e.g. in the form of tablets, coated tablets, dragées, hard and soft gelatine capsules, solutions, emulsions or suspensions, rectally, e.g. in the form of suppositories, or parentally, e.g. in the form of injection solutions.

The production of the pharmaceutical preparations can be affected in a manner which will be familiar to any person skilled in the art by bringing the substances in accordance with the invention, optionally in combination with other therapeutically valuable substances, into a galenical administration form together with suitable, non-toxic, inert therapeutically compatible solid or liquid carrier materials and, if desired, the usual pharmaceutical adjuvants.

Both inorganic and organic carrier materials are suitable as such carrier materials. Thus, for example, lactose, corn starch or derivatives thereof, talc, stearic acid or its salts can be used as carriers for tablets, coated tablets, dragées and hard gelatine capsules. Suitable carriers for soft gelatine capsules are, for example, vegetable oils, waxes, fats and semi-solid and liquid polyols (depending on the nature of the active ingredient no carriers are, however, required in the case of soft gelatine capsules). Suitable carrier materials for the production of solutions and syrups are, for example, water, polyols, sucrose, invert sugar and glucose. Suitable carrier materials for injection solutions are, for example, water, alcohols, polyols, glycerol and vegetable oils. Suitable carrier materials for suppositories are, for example, natural or hardened oils, waxes, fats and semi-liquid or liquid polyols.

The usual preservatives, solubilizers, stabilizers, wetting agents, emulsifiers, sweeteners, colorants, flavorants, salts for varying the osmotic pressure, buffers, masking agents and antioxidants come into consideration as pharmaceutical adjuvants. For parenteral administration the compounds of formula I and, respectively, their salts are preferably provided as lyophilizates or dry powders for dilution with usual carriers such as water or isotonic saline.

As mentioned earlier, the compounds of formula I and their salts have antibacterial activity. They inhibit bacterial dihydrofolate reductase and potentiate the antibacterial activity of sulphonamides, such as e.g. sulfisoxazole, sulfadimethoxine, sulfamethoxazole, 4-sulphanilamido-5,6-dimethoxypyrimidine, 2-sulphanilamido-4,5-dimethylpyrimidine or sulfaquinoxaline, sulfadiazine, sulfamonomethoxine, 2-sulphanilamido-4,5-dimethylisoxazole and other inhibitors of enzymes which are involved in folic acid biosynthesis, such as e.g. pteridine derivatives.

Oral, rectal and parenteral administration come into consideration in human medicine for such combinations of one or more compounds I in accordance with the invention with sulphonamides. The ratio of compound I to sulphonamide can vary within a broad range; it amounts to e.g. between 1:40 (parts by weight) and 1:1 (parts by weight); preferred ratios are 1:10 to 1:2.

Thus, e.g., a tablet can contain 80 mg of a compound I in accordance with the invention and 400 mg of sulfamethoxazole, a paediatric tablet can contain 20 mg of a compound I in accordance with the invention and 100 mg of sulfamethoxazole; syrup per 5 ml can contain 40 mg of compound I and 200 mg of sulfamethoxazole.

A daily dosage of about 0.2 g to about 2 g of a compound of formula I in accordance with the invention comes into consideration for adults.

The compounds of formula I are characterized by a high antibacterial activity and a pronounced synergetic affect in combination with sulphonamides and are well tolerated.

The following Examples illustrate the invention in more detail.

Example 1

1.1. Preparation of (E)-(RS)-3-[5-(2,4-diamino-pyrimidin-5-ylmethyl)-2,3-dimethoxy-phenyl]-1-(1-phenyl-1H-phthalazin-2-yl)-propenone

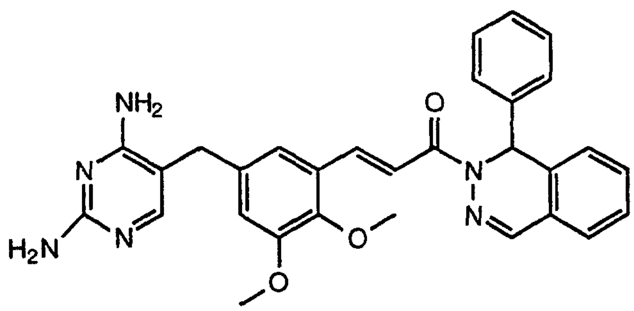

A solution 1.25 g of 5-(3-iodo-4,5-dimethoxy-benzyl)-pyrimidine-2,4-diamine and 45 mg of bis-(triphenylphosphine)-palladium(II) dichloride in 18 ml of N,N-dimethylformamide is heated to 120°C and treated dropwise at this temperature with a solution of 850 mg of (RS)-1-(1-phenyl-1H-phthalazin-2-yl)-propenone in 7 ml of N,N-dimethylformamide and 4.5 ml of triethylamine. After a reaction period of 30 min. at 120°C the dark mixture is concentrated on a rotary evaporator and the residue is chromatographed on 130 g of silica gel (eluent: methylene chloride/methanol/25% ammonia 95:5:0.5 v/v). The pure fractions are combined, concentrated and reprecipitated from ethyl acetate/hexane. 316 mg (19%) of (E)-(RS)-3-[5-(2,4-diamino-pyrimidin-5-ylmethyl)-2,3-dimethoxy-phenyl]-1-(1-phenyl-1H-phthalazin-2-yl)-propenone were isolated as a beige product, m.p. 130°C.

The following compounds were prepared in analogy to Example 1.1.:

1.2. (E)-(RS)-3-[5-(2,4-Diamino-pyrimidin-5-ylmethyl)-2,3-dimethoxyphenyl]1-(1-pyridin-2-yl-1H-phthalazin-2-yl)-propenone, m.p. 146°C.

1.3. (E)-(RS)-1-(1-Butyl-1H-phthalazin-2-yl)-3-[5-(2,4-diamino-pyrimidin-5-ylmethyl)-2,3-dimethoxy-phenyl]-propenone, m.p. 110°C, dec.

1.4. (E)-(RS)-3-[5-(2,4-Diamino-pyrimidin-5-ylmethyl)-2,3-dimethoxyphenyl]-1-(1-furan-2-yl-1H-phthalazin-2-yl)-propenone, m.p. 140°C.

1.5. (E)-(RS)-3-[5-(2,4-Diamino-pyrimidin-5-ylmethyl)-2,3-dimethoxyphenyl]-1-(1-thiazol-2-yl-1H-phthalazin-2-yl)-propenone, m.p. 145°C.

1.6. (E)-(RS)-2-(2-{3-[5-(2,4-Diamino-pyrimidin-5-ylmethyl)-2,3-dimethoxyphenyl]-acryloyl}-1,2-dihydro-phthalazin-1-yl)-N,N-dimethyl-benzene-suphonamide, m.p. 160°C.

1.7. (E)-(RS)-3-[5-(2,4-Diamino-pyrimidin-5-ylmethyl)-2,3-dimethoxyphenyl]-1-[1-(2-dimethylaminomethyl-phenyl)-1H-phthalazin-2-yl]-propenone, m.p. 106°C, dec.

1.8. (E)-(RS)-3-[5-(2,4-Diamino-pyrimidin-5-ylmethyl)-2,3-dimethoxyphenyl]-1-[1-(5-methyl-pyridin-2-yl)-1H-phthalazin-2-yl]-propenone, MS (ISP): 536 $(M+H)^+$.

1.9. (E)-(RS)-2-(2-{3-[5-(2,4-Diamino-pyrimidin-5-ylmethyl)-2,3-dimethoxyphenyl]-acryloyl}-1,2-dihydro-phthalazin-1-yl)-cyclopentanone (mixture of diastereomers), MS (ISP): 527 $(M+H)^+$.

1.10. (E)-(RS)-2-(2-{3-[5-(2,4-Diamino-pyrimidin-5-ylmethyl)-2,3-dimethoxyphenyl]-acryloyl}-1,2-dihydro-phthalazin-1-yl)-cyclohexanone (mixture of diastereomers) , MS (ISP): 541 $(M+H)^+$.

1.11. (E)-(RS)-3-[5-(2,4-Diamino-pyrimidin-5-ylmethyl)-2,3-dimethoxyphenyl]-1-[1-(2-oxo-propyl)-1H-phthalazin-2-yl]-propenone, MS (ISP): 501 $(M+H)^+$.

1.12. Methyl (E)-(RS)-2-(2-{3-[5-(2,4-Diamino-pyrimidin-5-ylmethyl)-2,3-dimethoxy-phenyl]-acryloyl}-1,2-dihydro-phthalazin-1-yl)-2-methyl-propionate, MS (ISP): 545 $(M+H)^+$.

1.13. (E)-(RS)-3-[5-(2,4-Diamino-pyrimidin-5-ylmethyl)-2,3-dimethoxyphenyl]-1-[1-(2-oxo-2-phenyl-ethyl)-1H-phthalazin-2-yl]-propenone, MS (ISP): 563 $(M+H)^+$.

1.14. (E)-(RS)-3-(2-{3-[5-(2,4-Diamino-pyrimidin-5-ylmethyl)-2,3-dimethoxyphenyl]-acryloyl}-1,2-dihydro-phthalazin-1-yl)-tetrahydro-pyran-2-one (mixture of diastereomers), MS (ISP): 543 $(M+H)^+$.

1.15. (E)-(RS)-3-[5-(2,4-dDiamino-pyrimidin-5-ylmethyl)-2,3-dimethoxyphenyl]-1-[1-(1-methyl-2-oxo-2-phenyl-ethyl)-1H-phthalazin-2-yl]-propenone (mixture of diastereomers), MS (ISP): 577 $(M+H)^+$.

1.16. (E)-(RS)-3-[5-(2,4-Diamino-pyrimidin-5-ylmethyl)-2,3-dimethoxyphenyl]-1-[1-(2-furan-2-yl-2-oxo-ethyl)-1H-phthalazin-2-yl]-propenone, MS (ISP): 553 $(M+H)^+$.

1.17.(E)-(RS)- 3-[5-(2,4-Diamino-pyrimidin-5-ylmethyl)-2,3-dimethoxyphenyl]-1-(1-methylsulfanylmethyl-1H-phthalazin-2-yl)-propenone, m.p. = 205°.

1.18.(E)-(RS)- 1-[1-(4-tert-Butyl-phenyl)-1H-phthalazin-2-yl]-3-[5-(2,4-diamino-pyrimidin-5-ylmethyl)-2,3-dimethoxy-phenyl]-propenone, m.p. = 123°.

1.19.(E)-(RS)-3-[5-(2,4-Diamino-pyrimidin-5-ylmethyl)-2,3-dimethoxyphenyl]-1-(1-p-tolyl-1H-phthalazin-2-yl)-propenone, m.p. = 145°.

1.20. (E)-(RS)- 3-[5-(2,4-Diamino-pyrimidin-5-ylmethyl)-2,3-dimethoxyphenyl]-1-[1-(4-ethyl-phenyl)-1H-phthalazin-2-yl]-propenone, m.p. = 107°.

1.21. (E)-(RS)- 3-[5-(2,4-Diamino-pyrimidin-5-ylmethyl)-2,3-dimethoxyphenyl]-1-[1-(4-trifluoromethoxy-phenyl)-1H-phthalazin-2-yl]-propenone, m.p. = 127°.

1.22.(E)-(RS)-3-[5-(2,4-Diamino-pyrimidin-5-ylmethyl)-2,3-dimethoxyphenyl]-1-[1-(3,4-dimethyl-phenyl)-1H-phthalazin-2-yl]-propenone, m.p. =139°.

1.23.(E)-(RS)-1-(1-tert-Butyl-1H-phthalazin-2-yl)-3-[5-(2,4-diaminopyrimidin-5-ylmethyl)-2,3-dimethoxy-phenyl]-propenone, m.p. =90°.

1.24. (E)-(RS)- 3-[5-(2,4-Diamino-pyrimidin-5-ylmethyl)-2,3-dimethoxyphenyl]-1-[1-(5,6-dihydro-4H-pyran-2-yl)-1H-phthalazin-2-yl]-propenone, MS (ISP): 527.3 $(M+H)^+$.

1.25.(E)-(RS)-3-[5-(2,4-Diamino-pyrimidin-5-ylmethyl)-2,3-dimethoxyphenyl]-1-[1-(2,4,6-trimethyl-phenyl)-1H-phthalazin-2-yl]-propenone, MS (ISP): 563.5 $(M+H)^+$.

1.26.(E)-R-(-)-3-[5-(2,4-Diamino-pyrimidin-5-ylmethyl)-2,3-dimethoxyphenyl]-1-(1-phenyl-1H-phthalazin-2-yl)-propenone; $[\alpha]_D$ = -661° (c=1; methanol).

1.27.(E)-S-(+)-3-[5-(2,4-Diamino-pyrimidin-5-ylmethyl)-2,3-dimethoxyphenyl)-1-(1-phenyl-1H-phthalazin-2-yl)-propenone; $[\alpha]_D$ = +664° (c=1 ; methanol).

1.28.(E)-S-(-)-3-[5-(2,4-Diamino-pyrimidin-5-ylmethyl)-2,3-dimethoxyphenyl]-1-(1-furan-2yl-1H-phthalazin-2-yl)-propenone; $[\alpha]_D$ = -543° (c=1; methanol).

1.29.(E)-R-(+)-3-[5-(2,4-Diamino-pyrimidin-5-ylmethyl)-2,3-dimethoxyphenyl]-1-(1-furan-2yl-1H-phthalazin-2-yl)-propenone; $[\alpha]_D$ = +580° (c=1; methanol).

1.30.(E)-R-(-)-3-[5-(2,4-Diamino-pyrimidin-5-ylmethyl)-2,3-dimethoxyphenyl]-1-(1-propyl-1H-phthalazin-2-yl)-propenone; $[\alpha]_D$ = -739° (c=1 ; methanol).

1.31.(E)-S-(+)-3-[5-(2,4-Diamino-pyrimidin-5-ylmethyl)-2,3-dimethoxyphenyl]-1-(1-propyl-1H-phthalazin-2-yl)-propenone; $[\alpha]_D$ = +790° (c=1 ; methanol).

1.32.(E)-R-(-)-3-[5-(2,4-Diamino-pyrimidin-5-ylmethyl)-2,3-dimethoxyphenyl]-1-(1-methyl-1H-phthalazin-2-yl)-propenone; $[\alpha]_D$ = -724° (c=1 ; methanol).

1.33.(E)-S-(+)-3-[5-(2,4-Diamino-pyrimidin-5-ylmethyl)-2,3-dimethoxyphenyl]-1-(1-methyl-1H-phthalazin-2-yl)-propenone; $[\alpha]_D$ = +689° (c=1 ; methanol).

1.34 (E)-(RS)-1-(1-Biphenyl-4-yl-1H-phthalazin-2-yl)-3-[5-(2,4-diaminopyrimidin-5-ylmethyl)-2,3-dimethoxy-phenyl]-propenone, m.p. 147°C (acetonitrile). MS (ISP): 597.3 $(M+H)^+$.

1.35 Mixture of (E)-3-[5-(2,4-Diamino-pyrimidin-5-ylmethyl)-2,3-dimethoxyphenyl]-1-[(RS)- und -[(SR)-1-[4-[(RS)-1-ethoxy-ethoxy]-phenyl]-1H-phthalazin-2-yl]-propenone, m.p. 130°C (ethanol). MS (ISP): 609.3 $(M+H)^+$.

1.36 (E)-1-[(RS)-1-Bicyclo[2.2.1]hept-2endo- and/or 2exo-yl-1H-phthalazin-2-yl]-3-[5-(2,4-diamino-pyrimidin-5-ylmethyl)-2,3-dimethoxy-phenyl]-propenone, m.p. 113-116°C (methanol). MS (ISP): 539.4 $(M+H)^+$.

1.37 (E)-(RS)-3-[5-(2,4-Diamino-pyrimidin-5-ylmethyl)-2,3-dimethoxyphenyl]-1-[1-(6-methyl-pyridin-2-yl)-1H-phthalazin-2-yl]-propenone, m.p. 134-138°C (ethanol). MS (ISP): 536.3 $(M+H)^+$.

1.38 (E)-(RS)-3-[5-(2,4-Diamino-pyrimidin-5-ylmethyl)-2,3-dimethoxyphenyl]-1-[1-(4-dimethylamino-phenyl)-1H-phthalazin-2-yl]-propenone, m.p. 270-275°C (acetonitrile/methanol). MS (ISP): 564.4 $(M+H)^+$.

1.39 (E)-(RS)-3-[5-(2,4-Diamino-pyrimidin-5-ylmethyl)-2,3-dimethoxyphenyl]-1-[1-[4-(2-ethoxy-ethoxy)-phenyl]-1H-phthalazin-2-yl]-propenone, m.p. 111-115°C (ethanol). MS (ISP): 609.3 $(M+H)^+$.

1.40 (E)-(RS)-3-[5-(2,4-Diamino-pyrimidin-5-ylmethyl)-2,3-dimethoxyphenyl]-1-(1-m-tolyl-1H-phthalazin-2-yl)-propenone, m.p. 134-139°C (methanol). MS (ISP): 535.4 $(M+H)^+$.

1.41 (E)-(RS)-3-[5-(2,4-Diamino-pyrimidin-5-ylmethyl)-2,3-dimethoxyphenyl]-1-[1-(3,5-dimethyl-phenyl)-1H-phthalazin-2-yl]-propenone, m.p. 134-137°C (methanol). MS (ISP): 549.3 $(M+H)^+$.

1.42.(E)-(RS)-3-[5-(2,4-Diamino-pyrimidin-5-ylmethyl)-2,3-dimethoxyphenyl]-1-[1-(4-pyrrol-1-yl-phenyl)-1H-phthalazin-2-yl]-propenone, m.p. 137-143°C (ethanol). MS (ISP): 586.4 $(M+H)^+$.

1.43.(E)-(R)-3-[5-(2,4-Diamino-pyrimidin-5-ylmethyl)-2,3-dimethoxy-phenyl]-1-[1-(6-methyl-pyridin-3-yl)-1H-phthalazin-2-yl]-propenone, m.p. 140-143°C (acetonitrile), $[\alpha]_D$= +614° (c=1, chloroform). MS (ISP): 536.4 $(M+H)^+$.

1.44 (E)-(S)-3-[5-(2,4-Diamino-pyrimidin-5-ylmethyl)-2,3-dimethoxy-phenyl]-1-[1-(6-methyl-pyridin-3-yl)-1H-phthalazin-2-yl]-propenone, m.p. 139-19:2°C (acetonitrile/ethanol), $[\alpha]_D$= -626° (c=1, chloroform). MS (ISP): 536.4

(M+H)+.

1.45 (E)-(RS)-3-[5-(2,4-Diamino-pyrimidin-5-ylmethyl)-2,3-dimethoxyphenyl]-1-[1-(2,3-dihydro-benzo[1,4]dioxin-6-yl)-1H-phthalazin-2-yl]-propenone, m.p. 212-215°C (methanol). MS (ISP): 579.2 $(M+H)^+$.

1.46 (E)-(RS)-1-(1-Cyclopropyl-1H-phthalazin-2-yl)-3-[5-(2,4-diaminopyrimidin-5-ylmethyl)-2,3-dimethoxy-phenyl] -propenone, m.p. 230-236°C (acetonitrile/ethanol). MS (ISP): 485.4 $(M+H)^+$.

1.47 (E)-(RS)-3-[5-(2,4-Diamino-pyrimidin-5-ylmethyl)-2,3-dimethoxyphenyl]-1-[1-(4-methylsulfanyl-phenyl)-1H-phthalazin-2-yl]-propenone, m.p. 168-172°C (acetonitrile). MS (ISP): 567.6 $(M+H)^+$.

1.48 (E)-(RS)-3-[5-(2,4-Diamino-pyrimidin-5-ylmethyl)-2,3-dimethoxyphenyl]-1-[1-(6-methyl-pyridin-3-yl)-1H-phthalazin-2-yl]-propenone, m.p. 153-156°C (ethanol). MS (ISP): 536.4 $(M+H)^+$.

1.49 (E)-(RS)-3-[5-(2,4-Diamino-pyrimidin-5-ylmethyl)-2,3-dimethoxyphenyl]-1-(1-[1,3]dithian-2-yl-1H-phthalazin-2-yl)-propenone, m.p. 235-240°C (acetonitrile). MS (ISP): 563.4 $(M+H)^+$.

1.50 (E)-(RS)-3-[5-(2,4-Diamino-pyrimidin-5-ylmethyl)-2,3-dimethoxyphenyl]-1-[1-(6-dimethylamino-pyridin-3-yl)-1H-phthalazin-2-yl]-propenone, m.p. 128-132°C (methanol). MS (ISP): 565.4 $(M+H)^+$.

1.51 (E)-(RS)-3-[5-(2,4-Diamino-pyrimidin-5-ylmethyl)-2,3-dimethoxyphenyl]-1-(1-pyridin-3-yl-1H-phthalazin-2-yl)-propenone, mp. 138-142°C (methanol), MS (ISP): 522.2 $(M+H)^+$.

1.52 (E)-(R)-1-(1-Cyclopropyl-1H-phthalazin-2-yl)-3-[5-(2,4-diaminopyrimidin-5-ylmethyl)-2,3-dimethoxy-phenyl]-propenone, m.p. 125-128°C (methanol), $[\alpha]_D$ = +845° (c=1, chloroform). MS (ISP): 485.3 $(M+H)^+$.

1.53 (E)-(S)-1-(1-Cyclopropyl-1H-phthalazin-2-yl)-3-[5-(2,4-diaminopyrimidin-5-ylmethyl)-2,3-dimethoxy-phenyl]-propenone, m.p. 123-130°C (methanol), $[\alpha]_D$ = -824° (c=0.1, chloroform). MS (ISP): 485.3 $(M+H)^+$.

1.54 (E)-(R)-3-[5-(2,4-Diamino-pyrimidin-5-ylmethyl)-2,3-dimethoxy-phenyl]-1-[1-(4-methylsulfanyl-phenyl)-1H-phthalazin-2-yl]-propenone, m.p. 139-143°C (acetonitril), $[\alpha]_D$= +581° (c=1, chloroform). MS (ISP): 567.3 $(M+H)^+$.

1.55 (E)-(S)-3-[5-(2,4-Diamino-pyrimidin-5-ylmethyl)-2,3-dimethoxy-phenyl]-1-[1-(4-methylsulfanyl-phenyl)-1H-phthalazin-2-yl]-propenone, m.p. 137-142°C (acetonitrile), $[\alpha]_D$ = -590° (c=1, chloroform). MS (ISP): 567.4 $(M+H)^+$.

1.56 (E)-(RS)-1-(1-Cyclobutyl-1H-phthalazin-2-yl)-3-[5-(2,4-diaminopyrimidin-5-ylmethyl)-2,3-dimethoxy-phenyl]-propenone, m.p. 122-129°C (methanol), MS (ISP): 499.3 $(M+H)^+$.

1.57 (E)-(RS)-1-(1-Cyclopentyl-1H-phthalazin-2-yl)-3-[5-(2,4-diaminopyrimidin-5-ylmethyl)-2,3-dimethoxy-phenyl]-propenone, m.p. 118-123°C (methanol). MS (ISP): 513.4 $(M+H)^+$.

1.58 (E)-(RS)-3-[5-(2,4-Diamino-pyrimidin-5-ylmethyl)-2,3-dimethoxyphenyl]-1-[1-(4-trifluoromethyl-phenyl)-1H-phthalazin-2-yl]-propenone, m.p. 189-192°C (methanol). MS (ISP): 589.3 $(M+H)^+$.

1.59 (E)-(RS)-1-[1-(5-Chloro-pyridin-2-yl)-1H-phthalazin-2-yl]-3-[5-(2,4-diamino-pyrimidin-5-ylmethyl)-2,3-dimethoxy-phenyl]-propenone, m.p. 133°C (methanol). MS (ISP): 556.2 $(M+H)^+$.

1.60 (E)-(RS)-1-(1-Cyclohexyl-1H-phthalazin-2-yl)-3-[5-(2,4-diaminopyrimidin-5-ylmethyl)-2,3-dimethoxy-phenyl]-propenone, m.p. 120°C (methanol). MS (ISP): 527.3 $(M+H)^+$.

1.61 (E)-(RS)-3-[5-(2,4-Diamino-pyrimidin-5-ylmethyl)-2,3-dimethoxyphenyl]-1-[1-(4-methoxy-phenyl)-1H-phthalazin-2-yl]-propenone, m.p. 199-201°C (methanol). MS (ISP): 551.2 $(M+H)^+$.

1.62 (E)-(RS)-3-[5-(2,4-Diamino-pyrimidin-5-ylmethyl)-2,3-dimethoxyphenyl]-1-[1-(3,4-dimethoxy-phenyl)-1H-phthalazin-2-yl]-propenone, m.p. 155°C (methanol). MS (ISP): 581.3 $(M+H)^+$.

1.63 (E)-(RS)-1-(1-Benzo[1,3]dioxol-5-yl-1H-phthalazin-2-yl)-3-[5-(2,4-diaminopyrimidin-5-ylmethyl)-2,3-dimethoxy-phenyl]-propenone, m.p. 131-136°C (methanol). MS (ISP): 565.3 $(M+H)^+$.

1.64 (E)-(RS)-4-[2-[3-[5-(2,4-Diamino-pyrimidin-5-ylmethyl)-2,3-dimethoxyphenyl] -acryloyl]-1,2-dihydro-phthalazin- 1-yl] -benzonitril, m.p. 138-146°C (methanol). MS (ISP): 546.2 $(M+H)^+$.

1.65 (E)-(RS)-4-[2-[3-[5-(2,4-Diamino-pyrimidin-5-ylmethyl)-2,3-dimethoxyphenyl]-acryloyl]-1,2-dihydro-phthalazin-1-yl]-benzoic acid tert-butyl ester, m.p. 165-169°C (methanol). MS (ISP): 621.5 $(M+H)^+$.

1.66 (E)-(RS)-3-[5-(2,4-Diamino-pyrimidin-5-ylmethyl)-2,3-dimethoxyphenyl]-1-(1-thiophen-2-yl-1H-phthalazin-2-yl)-propenone, m.p. 150-155°C (ethanol). MS (ISP): 527.3 $(M+H)^+$.

1.67 (E)-(RS)-1-[1-(5-Butyl-thiophen-2-yl)-1H-phthalazin-2-yl]-3-[5-(2,4-diamino-pyrimidin-5-ylmethyl)-2,3-dimethoxy-phenyl]-propenone, m.p. 107-111°C (methanol). MS (ISP): 583.3 $(M+H)^+$.

1.68 mixture of (E)-3-[5-(2,4-diamino-pyrimidin-5-ylmethyl)-2,3-dimethoxyphenyl]-1-[(RS)- and -[(SR)-1-[4-[(RS)-1-hydroxy-ethyl]-phenyl]-1H-phthalazin-2-yl]-propenone, m.p. 225-230°C (methanol). MS (ISP): 565.4 $(M+H)^+$.

1.69 (E)-(RS)-1-(1-Benzenesulfonylmethyl-1H-phthalazin-2-yl)-3-[5-(2,4-diamino-pyrimidin-5-ylmethyl)-2,3-dimethoxy-phenyl]-propenone, m.p. 128-133°C (methanol). MS (ISP): 599.2 $(M+H)^+$.

1.70 (E)-(RS)-4-[2-[3-[5-(2,4-Diamino-pyrimidin-5-ylmethyl)-2,3-dimethoxyphenyl]-acryloyl]-1,2-dihydro-phthalazin-1-yl]-N,N-dimethyl-benzenesulfonamid, m.p. 133-135°C (methylenchloride/methanol). MS (ISP): 628.3 $(M+H)^+$.

1.71 (E)-(RS)-3-[5-(2,4-Diamino-pyrimidin-5-ylmethyl)-2,3-dimethoxyphenyl]-1-[1-(4-methoxymethyl-phenyl)-1H-phthalazin-2-yl]-propenone, m.p. 165-167°C (methanol). MS (ISP): 565.4 $(M+H)^+$.

1.72 (E)-(RS)-3-[5-(2,4-Diamino-pyrimidin-5-ylmethyl)-2,3-dimethoxyphenyl]-1-[1-[4-[(diisopropylamino)-methyl]-phenyl]-1H-phthalazin-2-yl]-propenone, m.p. 139-144°C (methanol). MS (ISP): 634.4 $(M+H)^+$.

1.73 (E)-(RS)-1-[1-(4-Chloro-phenyl)-1H-phthalazin-2-yl]-3-[5-(2,4-diaminopyrimidin-5-ylmethyl)-2,3-dimethoxy-phenyl]-propenone, m.p. 158-160°C (acetonitrile). MS (ISP): 555.2 $(M+H)^+$.

1.74 (E)-(RS)-3-[5-(2,4-Diamino-pyrimidin-5-ylmethyl)-2,3-dimethoxyphenyl]-1-[1-(6-methoxy-pyridin-3-yl)-1H-phthalazin-2-yl]-propenone, m.p. 124-130°C (methanol). MS (ISP): 552.3 $(M+H)^+$.

1.75 (E)-(RS)-3-[5-(2,4-Diamino-pyrimidin-5-ylmethyl)-2,3-dimethoxyphenyl]-1-[1-[4-(4-methyl-piperazin-1-ylmethyl)-phenyl]-1H-phthalazin-2-yl]-propenone, m.p. 145°C. MS (ISP): 633.4 $(M+H)^+$.

1.76 (E)-(RS)-3-[5-(2,4-Diamino-pyrimidin-5-ylmethyl)-2,3-dimethoxyphenyl]-1-[1-(4-morpholin-4-ylmethyl-phenyl)-1H-phthalazin-2-yl]-propenone, m.p. 85-88°C (ethanol). MS (ISP): 620.4 $(M+H)^+$.

1.77 (E)-(RS)-1-(1-Adamantan-2-yl-1H-phthalazin-2-yl)-3-[5-(2,4-diaminopyrimidin-5-ylmethyl)-2,3-dimethoxy-phenyl]-propenone, m.p. 185-188°C (methylenchloride/methanol). MS (ISP): 579.3 $(M+H)^+$.

1.78 (E)-(RS)-3-[5-(2,4-Diamino-pyrimidin-5-ylmethyl)-2,3-dimethoxyphenyl]-1-{1-[6-(1-hydroxy-1-methyl-ethyl)-pyridin-3-yl]-1H-phthalazin-2-yl}-propenone, m.p. 147-152°C (ethanol). MS (ISP): 580.2 $(M+H)^+$.

1.79 (E)-(RS)-3-[5-(2,4-Diamino-pyrimidin-5-ylmethyl)-2,3-dimethoxyphenyl]-1-[1-(6-morpholin-4-yl-pyridin-3-yl)-1H-phthalazin-2-yl]-propenone, m.p. 142-148°C (ethanol). MS (ISP): 607.2 $(M+H)^+$.

1.80 (E)-(RS)-5-(2-{3-[5-(2,4-Diamino-pyrimidin-5-ylmethyl)-2,3-dimethoxyphenyl]-acryloyl}-1,2-dihydro-phthalazin-1-yl)-pyridin-2-carbonic acid tert-butylamide, m.p. 140-148°C (methylenchlorid/methanol). MS (ISP): 621.2 $(M+H)^+$.

Example 2

2.1. Preparation of (E)-(RS)-3-[5-(2,4-diamino-pyrimidin-5-ylmethyl)-2,3-dimethoxy-phenyl]-1-[1-(4-fluoro-phenyl)-1H-phthalazin-2-yl]-propenone

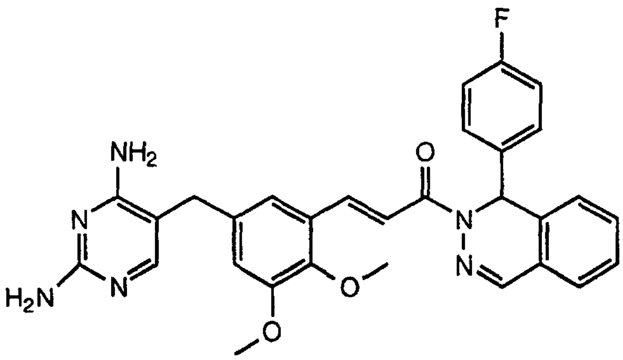

A solution of 0.819 g of 5-(3-iodo-4,5-dimethoxy-benzyl)-pyrimidin-2,4-diamine and 0.89 g of (RS)-1-[1-(4-fluoro-phenyl)-1H-phthalazin-2-yl]-propenone in 15 ml of N,N-dimethylacetamide and 1.9 ml of triethylamine is treated with 22 mg of palladium-(II) acetate and 127 mg of tri-o-tolylphosphine and heated to 120°C for 2.5 hrs. Subsequently, the hot orange solution is poured into 50 ml of aqueous saturated sodium hydrogen carbonate solution and stirred at room temperature for 15 min. The resulting precipitate is filtered off under suction dried and chromatographed over silica gel with dichloro-methane/methanol 95:5. The pure fractions are combined, concentrated and crystallized from dichloromethane/tert.butyl methyl ether. 603 mg (53%) of E)-(RS)-3-[5-(2,4-diamino-pyrimidin-5-ylmethyl)-2,3-dimethoxy-phenyl]-1-[1-(4-fluoro-phenyl)-1H-phthalazin-2-yl]-propenone are isolated as a colourless product, m.p. 114°C, dec.

The following compounds were prepared in analogy to Example 2.1.:

2.2. (E)-(RS)- 3-[5-(2,4-Diamino-pyrimidin-5-ylmethyl)-2,3-dimethoxyphenyl]-1-[1-(2,4-difluoro-phenyl)-1H-phthalazin-2-yl]-propenone, m.p. 121°C, dec.

2.3. (E)-(RS)- 3-[5-(2,4-Diamino-pyrimidin-5-ylmethyl)-2,3-dimethoxyphenyl]-1-(1-tridecafluorohexyl-1H-phthalazin-2-yl)-propenone, m.p. 143°C, dec.

2.4. (E)-3-[5-(2,4-Diamino-pyrimidin-5-ylmethyl)-2,3-dimethoxy-phenyl]-1-(1H-phthalazin-2-yl)-propenone, m.p. 164°C, dec.

2.5. (E)-(RS)-1-(1-Phenylsulphinylmethyl-1H-phthalazin-2-yl)-3-[5-(2,4-diamino-pyrimidin-5-ylmethyl)-2,3-dimethoxy-phenyl]-propenone, m.p. 135-138°C.

2.6. (E)-(RS)-3-{5-(2,4-Diamino-pyrimidin-5-ylmethyl)-2,3-dimethoxyphenyl}-1-{1-(4-hydroxymethyl-phenyl)-1H-phthalazin-2-yl}-propenone, m.p 265°C.

2.7. (E)-(RS)-3-{5-(2,4-Diamino-pyrimidin-5-ylmethyl)-2,3-dimethoxyphenyl}-1-{1-(4-hydroxy-phenyl)-1H-phthalazin-2-yl}-propenone, MS (ISP): 537.3 $(M+H)^+$

2.8. (E)-(RS)-3-{5-(2,4-Diamino-pyrimidin-5-ylmethyl)-2,3-dimethoxyphenyl}-1-{1-(2-hydroxymethyl-phenyl)-1H-phthalazin-2-yl}-propenone. M.p. 259°C

2.9. (E)-(RS)-3-{5-(2,4-Diamino-pyrimidin-5-ylmethyl)-2,3-dimethoxyphenyl}-1-{1-(3-hydroxymethyl-phenyl)-1H-phthalazin-2-yl}-propenone. M.p. 178-79°C.

2.10. (E)-(RS)-3-{5-(2,4-Diamino-pyrimidin-5-ylmethyl)-2,3-dimethoxyphenyl}-1-{1-(3-hydroxy-phenyl)-1H-phthalazin-2-yl}-propenone. M.p. 169-171°C

2.11. (E)-(RS)-3-{5-(2,4-Diamino-pyrimidin-5-ylmethyl)-2,3-dimethoxyphenyl}-1-(1-methyl-1H-phthalazin-2-yl)-propenone. M.p. 226°C

2.12.(E)-(RS)-3-{5-(2,4-Diamino-pyrimidin-5-ylmethyl)-2,3-dimethoxyphenyl}-1-{1-(4-(2-hydroxyethyl)-phenyl)-1H-phthalazin-2-yl}-propenone. M.p. 260°C.

2.13. (E)-(RS)-3-{5-(2,4-Diamino-pyrimidin-5-ylmethyl)-2,3-dimethoxyphenyl}-1-(1-ethyl-1H-phthalazin-2-yl)-propenone. MS (ISP): 473.4 $(M+H)^+$

2.14. (E)-(RS)-3-{5-(2,4-Diamino-pyrimidin-5-ylmethyl)-2,3-dimethoxyphenyl}-1-{1-(4-carbamoyloxymethyl-phenyl)-1H-phthalazin-2-yl}-propenone. MS (ISP): 594.4 $(M+H)^+$

2.15. (E)-(RS)-3-{5-(2,4-Diamino-pyrimidin-5-ylmethyl)-2,3-dimethoxyphenyl}-1-(1-propyl-1H-phthalazin-2-yl)-propenone. MS (ISP): 487.4 $(M+H)^+$

2.16. (E)-(RS)-3-{5-(2,4-Diamino-pyrimidin-5-ylmethyl)-2,3-dimethoxyphenyl}-1-(1-prop-2-yl-1H-phthalazin-2-yl)-propenone. MS (ISP): 487.4 $(M+H)^+$

2.17. (E)-(RS)-3-{5-(2,4-Diamino-pyrimidin-5-ylmethyl)-2,3-dimethoxyphenyl}-1-{1-(3-methyl-butyl)-1H-phthalazin-2-yl}-propenone. MS (ISP): 515.5 $(M+H)^+$

2.18. (E)-(RS)-3-{5-(2,4-Diamino-pyrimidin-5-ylmethyl)-2,3-dimethoxyphenyl}-1-(3-hydroxy-prop-1-yl-1H-phthalazin-2-yl)-propenone. MS (ISP): 503.3 $(M+H)^+$

2.19.(E)-(RS)-Morpholine-4-carboxylic acid 4-(2-{3-[5-(2,4-diamino-pyrimidin-5-ylmethyl)-2,3-dimethoxy-phenyl]-acryloyl}-1,2-dihydro-phthalazin-1-yl)-butyl ester. MS (ISP): 630.4 $(M+H)^+$

2.20.(E)-(RS)-3-{5-(2,4-Diamino-pyrimidin-5-ylmethyl)-2,3-dimethoxyphenyl}-1-{(4-hydroxy-but-1-yl)-1H-phthalazin-2-yl}-propenone. MS (ISP): 517.3 $(M+H)^+$

2.21.(E)-(RS)-Carbamic acid 4-(2-{3-[5-(2,4-diamino-pyrimidin-5-ylmethyl)-2,3-dimethoxy-phenyl]-acryloyl}-1,2-dihydro-phthalazin-1-yl)-butyl ester. MS(ISP): 560.3 $(M+H)^+$

2.22.(E)-(RS)-Carbamic acid 4-(2-{3-[5-(2,4-diamino-pyrimidin-5-ylmethyl)-2,3-dimethoxy-phenyl]-acryloyl}-1,2-dihydro-phthalazin-1-yl)-propyl ester. MS (ISP): 546.3 $(M+H)^+$

2.23.(E)-(RS)-3-[5-(2,4-Diamino-pyrimidin-5-ylmethyl)-2,3-dimethoxyphenyl]-1-[1-(2-[1,3]dioxolan-2-yl-ethyl)-1H-phthalazin-2-yl)-propenone. MS (ISP): 545.3 $(M+H)^+$

2.24.(E)-(RS)-1-Cyclohexylmethyl-1H-phthalazin-2-yl)-3-[5-(2,4-diaminopyrimidin-5-ylmethyl)-2,3-dimethoxy-phenylpropenone. MS (ISP): 541.4 $(M+H)^+$

Example 3

3.1. Preparation of (E)-(RS)-3-[5-(2,4-diamino-pyrimidin-5-ylmethyl)-3-methoxy-2-methoxymethoxy-phenyl]-1-(1-phenyl-1H-phthalazin-2-yl)-propenone

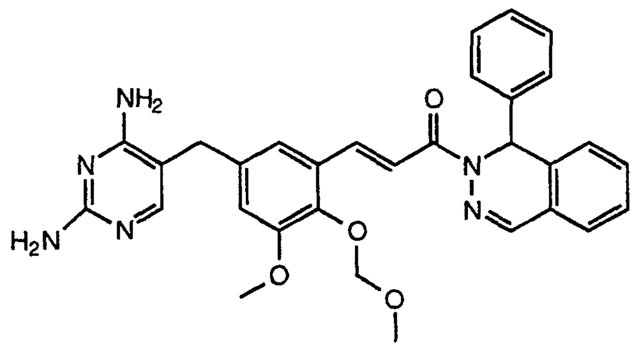

A solution of 806 mg of 5-(3-iodo-5-methoxy-4-methoxymethoxy-benzyl)-pyrimidine-2,4-diamine and 28 mg of bis-(triphenylphosphine)-palladium-(II) dichloride in 11 ml of N,N-dimethylformamide is heated to 120°C and treated dropwise at this temperature with a solution of 524 mg of (RS)-1-(1-phenyl-1H-phthalazin-2-yl)-propenone in 4 ml of N,N-dimethylformamide and 2.8 ml of triethylamine. After a reaction period of 3 hrs. at 120°C the dark mixture is concentrated and the residue is chromatographed on 330 g of silica gel (eluent: methylene chloride/methanol 1:1 v/v). The pure fractions are combined. After isolation by concentration there are obtained 500 mg of (E)-(RS)-3-[5-(2,4-diamino-pyrimidin-5-ylmethyl)-3-methoxy-2-methoxymethoxy-phenyl]-1-(1-phenyl-1H-phthalazin-2-yl)-propenone as a yellowish foam. MS (ISP): 551 $(M+H)^+$.

The following compounds were prepared in analogy to example 3:

3.2. (E)-(RS)-3-[5-(2,4-Diamino-pyrimidin-5-ylmethyl)-3-methoxy-2-methoxymethoxy-phenyl]-1-(1-furan-2-yl-1H-phthalazin-2-yl)-propenone , MS (ISP): 541 $(M+H)^+$.

3.3. (E)-(RS)-3-[5-(2,4-Diamino-pyrimidin-5-ylmethyl)-3-methoxy-2-methoxymethoxy-phenyl]-1-[1-(4-hydroxymethyl-phenyl)-1H-phthalazin-2-yl]-propenone, MS (ISP): 581 $(M+H)^+$.

Example 4

Preparation of (E)-(RS)-3-{5-(2,4-diamino-pyrimidin-5-ylmethyl)-2,3-dimethoxy-phenyl}-1-{1[2-(2,2-dimethyl-[1,3]-dioxolan-4-yl)-ethyl]-1H-phthalazin-2-yl}-propenone

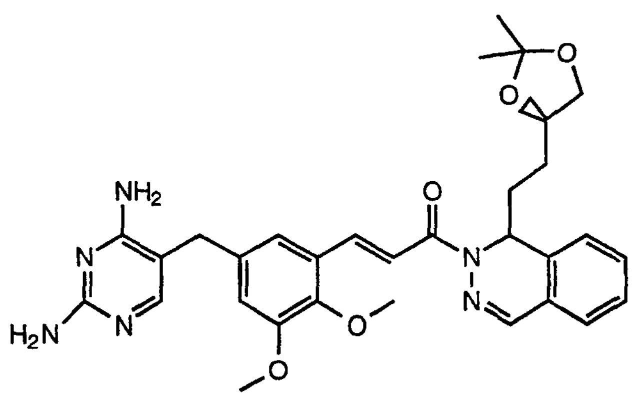

A solution of 1.3 g of 5-(3-iodo-4,5-dimethoxy-benzyl)-pyrimidin-2,4-diamine, 100 mg of bis-(triphenylphosphine)-palladium-(II) dichloride, 1.1 g of sodium bicarbonate, 4.7 ml of triethylamaine and 1.2 g of (E)-(RS)-1-{1 [2,2-dimethyl-[1,3]-dioxolan-4-yl]-ethyl}-1H-phthalazin-2-yl}-propenone in 25 ml of dimethylformamide is heated to 120°C. After 5 hrs. the pale yellow mixture is concentrated on a rotary evaporator and the residue is chromatographed on silica gel (eluent: $CH_2Cl_2$/MeOH/$NH_4OH$: 95/5/0.5). The pure fractions are combined, concentrated and crystallized from $CH_2Cl_2$. 720 mg of (E)-(RS)-3-{ 5-(2,4-diamino-pyrimidin-5-ylmethyl)-2,3-dimethoxy-phenyl}-1-{1[2-(2,2-dimethyl-[1,3]-dioxolan-4-yl)-ethyl]-1H-phthalazin-2-yl}-propenone are isolated as a beige product. MS (ISP): 573.5 $(M+H)^+$

Example 5

Preparation of (E)-(RS)-3-[5-(2,4-diamino-pyrimidin-5-ylmethyl)-3-methoxy-2-(2-morpholin-4-yl-ethoxy)-phenyl]-1-(1-phenyl-1H-phthalazin-2-yl)-propenone

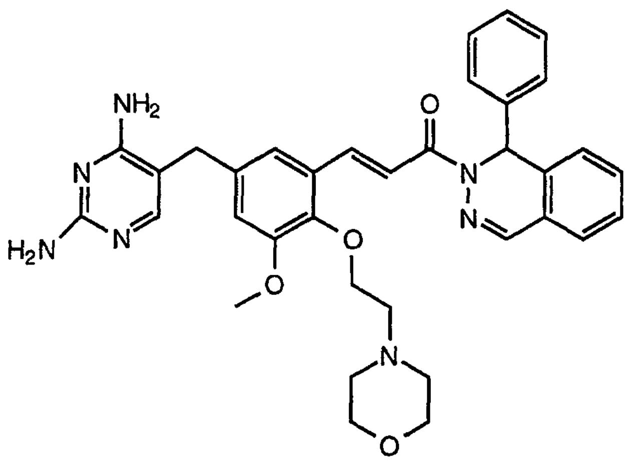

A solution of 120 mg of 5-{3-iodo-5-methoxy-4-(2-morpholin-4-yl-ethoxy)-benzyl}-pyrimidine-2,4-diamine and 28 mg of bis-(triphenylphosphine)-palladium-(II) dichloride in 1.5 ml of N,N-dimethylformamide is heated to 120°C and treated dropwise at this temperature with a solution of 76 mg of (RS)-1-(1-phenyl-1H-phthalazin-2-yl)-propenone in 0.75 ml of N,N-dimethyl-formamide and 0.75 ml of triethylamine. After 2.5 hrs. at 120°C the dark reaction mixture is concentrated and the residue is chromatographed on 50 g of silica gel (eluent, methylene chloride/methanol 1:1 v/v). The pure fractions are combined. 54 mg of (E)-(RS)-3-[5-(2,4-diamino-pyrimidin-5-ylmethyl)-3-methoxy-2-(2-morpholin-4-yl-ethoxy)-phenyl]-1-(1-phenyl-1H-phthalazin-2-yl)-propenone are isolated as a yellowish foam. MS (ISP): 620 $(M+H)^+$.

Example 6

Preparation of methyl (E)-(RS)-{4-(2,4-diamino-pyrimidin-5-ylmethyl)-2-methoxy-6-{3-oxo-3-(1-phenyl-1-H-phthalazin-2-yl)-propenyl}-phenoxy}-acetate

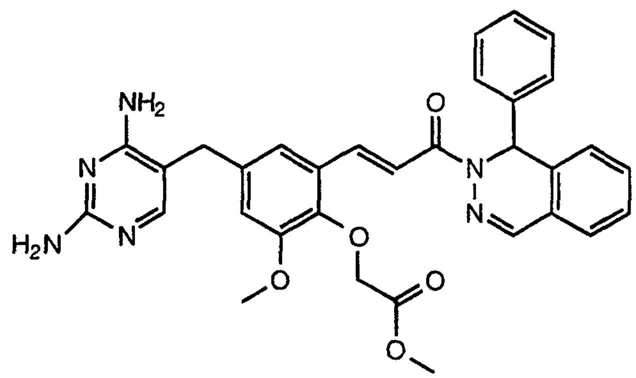

A solution of 380 mg of methyl {4-(2,4-diamino-pyrimidin-5-ylmethyl)-2-iodo-6- methoxy-phenoxy}-acetate and 96 mg of bis-(triphenylphosphine)-palladium-(II)dichloride in 5 ml of N,N-dimethylformamide is heated to 120°C and treated dropwise at this temperature with a solution of 250 mg of (RS)-1-(1-phenyl-1H-phthalazin-2-yl)-propenone in 2.5 ml of N,N-dimethyl-formamide and 2.5 ml of triethylamine. After 2.5 hrs. at 120°C the dark reaction mixture is concentrated and the residue is chromatographed on 100 g of silica gel (eluent: methylene chloride/methanol 1:1 v/v). The pure fractions are combined. 268 mg of methyl (E)-(RS)-{4-(2,4-diaminopyrimidin-5-ylmethyl)-2-methoxy-6-{3-oxo-3-(1-phenyl-1-H-phthalazin-2-yl)-propenyl}-phenoxy}-acetate are isolated as a yellowish foam. MS (ISP): 579 $(M+H)^+$.

Example 7

Preparation of (E)-(RS)-2-(2-{3-[5-(2,4-diamino-pyrimidin-5-ylmethyl)-2,3-dimethoxy-phenyl]-acryloyl}-1,2-dihydro-phthalazin-1-yl)-2-methyl-propionic acid

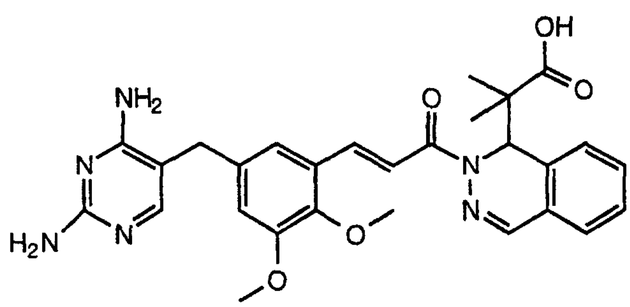

To the solution of 50 mg of methyl (E)-(RS)-2-(2-{3-[5-(2,4-diamino-pyrimidin-5-ylmethyl)-2,3-dimethoxy-phenyl]-acryloyl}-1,2-dihydro-phthalazin-1-yl)-2-methyl-propionate (Example 1.12) in 5 ml of methanol are added 3 drops of 2N sodium hydroxide solution and the mixture is held at reflux for 45 min. Subsequently, the mixture is concentrated, the residue is treated with ice-water and neutralized with 2N hydrochloric acid. The insoluble material is recrystallized from methylene/methanol. 41 mg of (E)-(RS)-2-(2-{3-[5-(2,4-diamino-pyrimidin-5-ylmethyl)-2,3-dimethoxy-phenyl]-acryloyl}-1,2-dihydro-phthalazin-1-yl)-2-methyl-propionic acid are isolated as a colourless solid. M.p = 245°C, dec.

Example 8

8.1. Preparation of (E)-(RS)-3-[5-(2,4-diamino-pyrimidin-5-ylmethyl)-2-hydroxy-3-methoxy-phenyl]-1-(1-phenyl-1H-phthalazin-2-yl)-propenone

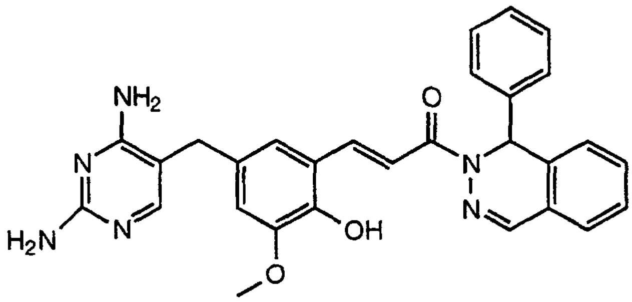

290 mg of (E)-(RS)-3-[5-(2,4-diamino-pyrimidin-5-ylmethyl)-3-methoxy-2-methoxymethoxy-phenyl]-1-(1-phenyl-1H-phthalazin-2-yl)-propenone;

Example 3.1. are dissolved in 10 ml of methanol and treated with 10 drops of conc. hydrochloric acid. After one hour at 70°C the mixture is concentrated and the residue is chromatographed on 250 g of silica gel (eluent: methylene chloride/methanol). 228 mg of (E)-(RS)-3-[5-(2,4-diamino-pyrimidin-5-ylmethyl)-2-hydroxy-3-methoxy-phenyl]-1-(1-phenyl-1H-phthalazin-2-yl)-propenone are isolated as an orange, hygroscopic solid. MS (ISP): $(M+H)^+$

The following compounds were prepared in analogy [to] 8.1.:

8.2. (E)-(RS)-3-[5-(2,4-Diamino-pyrimidin-5-ylmethyl)-2-hydroxy-3-methoxyphenyl]-1-(1-furan-2-yl-1H-phthalazin-2-yl)-propenone, m.p. 158°C, dec.

8.3. (E)-(RS)-3-[5-(2,4-Diamino-pyrimidin-5-ylmethyl)-2-hydroxy-3-methoxyphenyl]-1-[1-(4-hydroxymethyl-phenyl)-1H-phthalazin-2-yl]-propenone, m.p. > 250°C.

Example 9

9.1. Preparation of 2-amino-propionic acid 4-(2-{3-[5-(2,4-diamino-pyrimidin-5-ylmethyl)-2,3-dimethoxy-phenyl]-acryloyl}-1,2-dihydro-phthalazin-1-yl)-benzyl ester

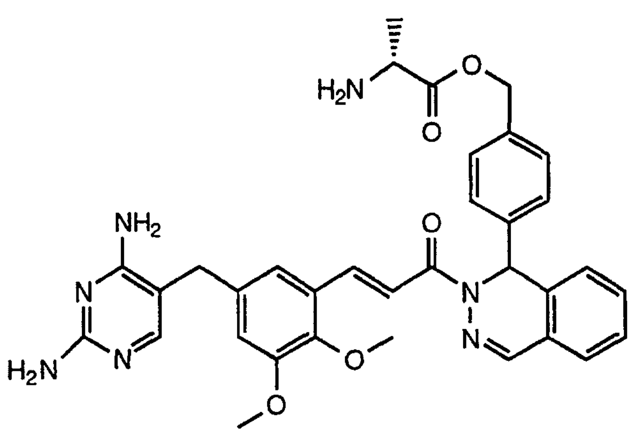

A solution of 130 mg of 2-tert-butoxycarbonylamino-propionic acid 4-(2-{3-[5-(2,4-diamino-pyrimidin-5-ylmethyl)-2,3-dimethoxy-phenyl] -acryloyl}-1,2-dihydro-phthalazin-1-yl)-benzyl ester in 20 ml of trifluoroethanol is treated

with 18 µl of methanesulphonic acid at room temperature over one hour. The reaction solution is concentrated, the residue is taken up in ethyl acetate and filtered. The crystals are dissolved in 5 ml of water and treated with 20 ml of pH 7 buffer. The suspension obtained is suction filtered. There are obtained 65 mg of 2-amino-propionic acid 4-(2-{3-[5-(2,4-diamino-pyrimidin-5-ylmethyl)-2,3-dimethoxy-phenyl]-acryloyl}-1,2-dihydro-phthalazin-1-yl)-benzyl ester, MS (ISP): 622.3 $(M+H)^+$

The educt used in Example 9.1. is prepared as follows:

a) A solution of 275 mg of (E)-(RS)-3-{5-(2,4-diamino-pyrimidin-5-ylmethyl)-2,3-dimethoxy-phenyl}-1-{1-(4-hydroxymethyl-phenyl)-1H-phthalazin-2-yl}-propenone in 10 ml of dimethylformamide is treated at room temperature with 105 mg of BOC-L-alanine, 95 mg of N-(3-dimethylaminopropyl-N'-ethylcarbodiimide hydrochloride and 30 mg of 4-dimethyl aminopyridine. After one hour the reaction solution is concentrated and the residue is chromatographed on silica gel (eluent: dichloromethane/ ethanol 9/1). 220 mg (61%) of 2-tert-butoxycarbonylamino-propionic acid-4-(2-{3-[5-(2,4-diamino-pyrimidin-5-ylmethyl)-2,3-dimethoxy-phenyl]-acryloyl}-1,2-dihydrophthalazin-1-yl)-benzyl ester are obtained as a pale yellow solid, MS (ISP) 722.5 $(M+H)^+$

9.2. 2-Amino-5-guanidino-pentanoic acid 4-(2-{3-[5-(2,4-diamino-pyrimidin-5-ylmethyl)-2,3-dimethoxy-phenyl]-acryloyl}-1,2-dihydro-phthalazin-1-yl)-benzyl ester is prepared in analogy to Example 9.1. MS (ISP): 707.6 $(M+H)^+$

The educt used in Example 9.2 (old 1.3.2.) is prepared in analogy to Example 9.1.a) from a 1:1 mixture of (S)-5-(N,N'-bis-tert-butoxycarbonyl-guanidino)-2-tert-butoxycarbonylamino-pentanoic acid (E)-(R)-and -(S)-4-[2-[3-[5-(2,4-diamino-pyrimidin-5-ylmethyl)-2,3-dimethoxy-phenyl]-acryloyl]-1,2-dihydrophthalazin-1-yl]-benzyl ester. MS (ISP): 1007.9 $(M+H)^+$

<u>Example 10</u>

10.1. Preparation of sulphuric acid (E)-(RS)-mono-[4-[2-[3-[5-(2,4-diaminopyrimidin-5-ylmethyl)-2,3-dimethoxy-phenyl]-acryloyl]-1,2-dihydrophthalazin-1-yl]-benzyl ester] Na salt (1:1)

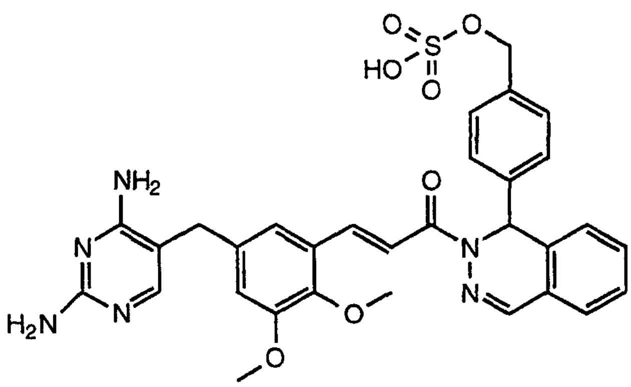

A solution 200 mg of (E)-(RS)-3-{ 5-(2,4-diamino-pyrimidin-5-ylmethyl)-2,3-dimethoxy-phenyl}-1-{1-(4-hydroxymethyl-phenyl)-1H-phthalazin-2-yl}-propenone in 5 ml of dimethylformamide is treated at room temperature with 67 mg of sulphur trioxide-dimethylformamide complex. After 1 hour 73 mg of sodium bicarbonate and 5 ml of water are added. The solution is stirred for a further hour, thereafter the solution is concentrated, the residue is taken up in 40 ml of methanol/dichloromethane and filtered. The mother liquor is concentrated and the residue is crystallized from methanol. There are obtained 90 mg of sulphuric acid (E)-(RS)-mono-[4-[2-[3-[5-(2,4-diamino-pyrimidin-5-ylmethyl)-2,3-dimethoxy-phenyl]-acryloyl]-1,2-dihydrophthalazin-1-yl]-benzyl ester] Na salt (1:1), MS (ISP): 631.3 $(M+H)^+$

10.2. Sulphuric acid (E)-(RS)-mono-[4-[2-[3-[5-(2,4-diamino-pyrimidin-5-ylmethyl)-2,3-dimethoxy-phenyl]-acryloyl]-1,2-dihydro-phthalazin-1-yl]-butyl ester] Na salt (1:1), MS (ISP): 597.3 $(M+H)^+$, is prepared in a analogous manner to Example 10.1.

Example 11

Preparation of (E)-(RS)-3-{ 5-(2,4-diamino-pyrimidin-5-ylmethyl)-2,3-dimethoxy-phenyl}-1-{1-(4-thiocyanato-butyl)-1H-phthalazin-2-yl}-propenone

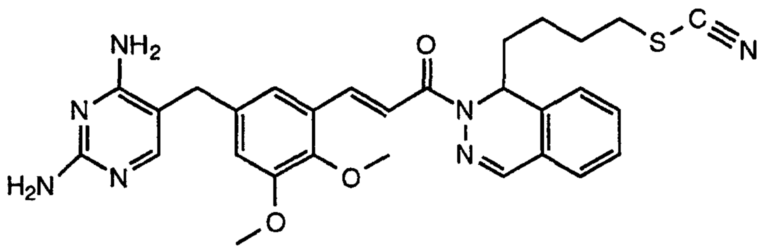

A solution of 206 mg of (E)-(RS)-3-{5-(2,4-diamino-pyrimidin-5-ylmethyl)-2,3-dimethoxy-phenyl}-1-{(4-hydroxy-but-1-yl)-1H-phthalazin-2-yl}-propenone in 2 ml of pyridine is treated with 37 µl of mesyl chloride at -20°C. After one hour the solution is concentrated and the residue, dissolved in 10 ml of dimethylformamide, is treated with 233 mg of potassium rhodanide at 80°C for 24 hrs. The reaction solution is concentrated and the residue is chromatographed on silica gel (eluent: $CH_2Cl_2$/MeOH/$NH_4OH$: 95/5/0.5). The pure fractions are combined, concentrated and crystallized from methanol. 124 mg of (E)-(RS)-3-{ 5-(2,4-diamino-pyrimidin-5-ylmethyl)-2,3-dimethoxy-phenyl}-1-{1-(4-thiocyanato-butyl)-1H-phthalazin-2-yl}-propenone are obtained as white cystals. MS (ISP): 558.3 $(M+H)^+$

Example 12

Preparation of (E)-(RS)-3-{ 5-(2,4-diamino-pyrimidin-5-ylmethyl)-2,3-dimethoxy-phenyl}-acryloyl-1,2-dihydrophthalazin-1-yl-propionaldehyde

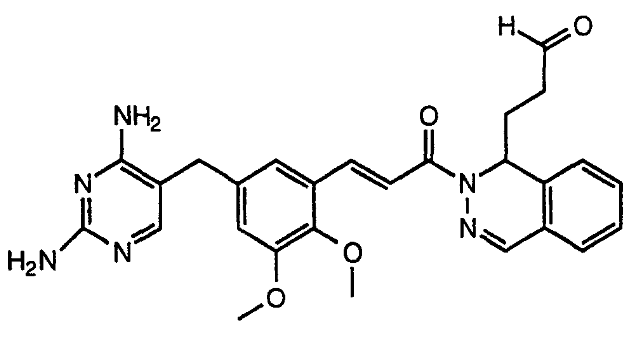

A solution of 764 mg of (E)-(RS)-3-[5-(2,4-diamino-pyrimidin-5-ylmethyl)-2,3-dimethoxy-phenyl]-1-[1-(2-[1,3] dioxolan-2-yl-ethyl)-1H-phthalazin-2-yl)-propenone in 20 ml of acetone and 3 ml of water is treated with 3 ml of 2N hydrochloric acid over two days. The pH is adjusted to pH 7-8 using sodium bicarbonate solution and the aqueous phase is extracted three times with dichloromethane/methanol (9:1). The organic phase is back-washed with sodium chloride solution, dried over magnesium sulphate, filtered and the filtrate is concentrated. The residue is chromatographed on silica gel (eluent: $CH_2Cl_2$/MeOH/ $NH_4OH$: 9/1/1). The pure fractions are combined, concentrated and crystallized from dichloromethane/hexane. The crystals are taken up in 150 ml of dichloromethane, stirred and suction filtered. The mother liquor is concentrated to about 25 ml and diluted with hexane. The crystals obtained are filtered off under suction. 388 mg of not quite pure (E)-(RS)-3-{5-(2,4-diamino-pyrimidin-5-ylmethyl)-2,3-dimethoxy-phenyl}-acryloyl-1,2-dihydrophthalazin-1-yl-propionaldehyde are obtained.

Example 13

13.1. Preparation of a mixture of (E) and (Z)-(RS)-5[2-[(E)-3-[5-(2,4-diamino-pyrimidin-5-ylmethyl)-2,3-dimethoxy-phenyl]-acryloyl]-1,2-dihydro-phthalazin-1-yl]-pent-2-enenitrile.

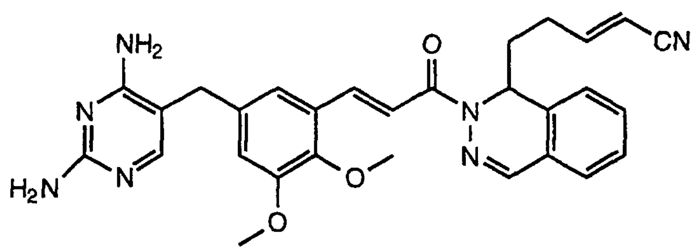

A suspension of 160 mg of (E)-(RS)-3-{5-(2,4-diamino-pyrimidin-5-ylmethyl)-2,3-dimethoxy-phenyl}-acryloyl-1,2-dihydrophthalazin-1-yl-propionaldehyde in 30 ml of tetrahydrofuran and 10 ml of dichloromethane is treated with 438 mg of cyanomethylene-triphenylphosphorane. After 12 hrs. the solution is concentrated and the residue is chromatographed on silica gel (eluent: $CH_2Cl_2$/MeOH/ $NH_4OH$: 95/5/0.5). The pure fractions are combined, concentrated and cystallized from $CH_2Cl_2$/MeOH. There are obtained 150 mg of white crystals. MS (ISP): 524.4 $(M+H)^+$

The following compounds were prepared analogously to Example 13.1:

13.2. Allyl (E)-(RS)-5[2-[(E)-3-[5-(2,4-diamino-pyrimidin-5-ylmethyl)-2,3-dimethoxy-phenyl]-acryloyl]-1,2-dihydrophthalazin-1-yl]-pent-2-enoate. MS-(ISP): 583.4 $(M+H)^+$

13.3.(EE)-(RS)-3-[3-(2-{3-[5-(2,4-Diamino-pyrimidin-5-ylmethyl)-2,3-dimethoxy-phenyl]-acryloyl}-1,2-dihydrophthalazin-1-yl]-propylidene]-1-hydroxy-pyrrolidin-2-one

Example 14

Preparation of 3-[3-(2-{3-[5-(2,4-diamino-pyrimidin-5-ylmethyl)-2,3-dimethoxy-phenyl]-acryloyl}-1,2-dihydrophthalazin-1-yl]-propylidene]-1-(2-trimethylsilanyl-ethoxy)-pyrrolidin-2-one.

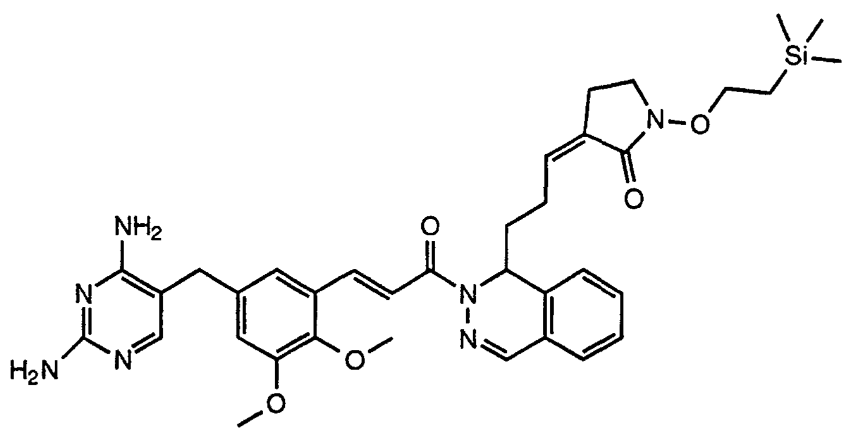

A mixture of 230 mg of (E)-(RS)-3-{ 5-(2,4-diamino-pyrimidin-5-ylmethyl)-2,3-dimethoxy-phenyl}-acryloyl-1,2-dihydrophthalazin-1-yl-propionaldehyde, 498 mg of (RS)-[2-oxo-1-(2-trimethylsilanyl-ethoxy)-pyrrolidin-3-yl]-triphenylphosphonium, 5 ml of butylene oxide and 5 ml of dichloromethane is heated to 50°C. After 8 hrs. the reaction solution is concentrated and the residue is chromatographed on silica gel (eluent: $CH_2Cl_2$/MeOH/$NH_4OH$: 95/5/0.5). The pure fractions are combined, concentrated and cystallized from $CH_2Cl_2$/hexane. There are obtained 222 mg of white crystals, MS (ISP): 684.5 $(M+H)^+$.

The educt used in this reaction can be prepared as described in EP-A 0 620 225.

Example 15

Preparation of 3-[3-(2-{3-[5-(2,4-diamino-pyrimidin-5-ylmethyl)-2,3-dimethoxyphenyl]-acryloyl}-1,2-dihydrophthalazin-1-yl]-propylidene]-1-hydroxy-pyrrolidin-2-one.

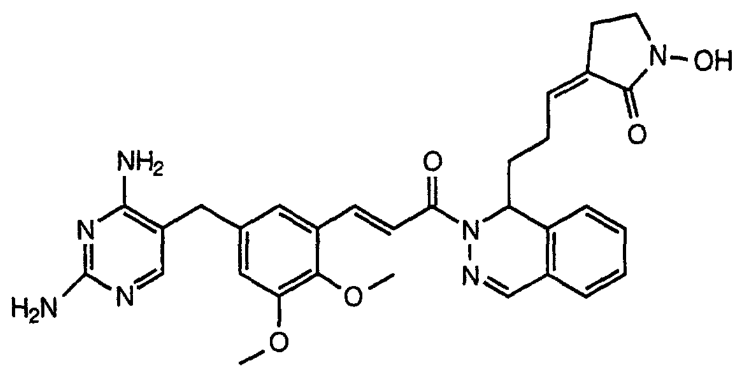

A solution of 220 mg of [3-(2-{3-[5-(2,4-diamino-pyrimidin-5-ylmethyl)-2,3-dimethoxy-phenyl]-acryloyl}-1,2-dihydrophthalazin-1-yl]-propylidene]-1-(2-trimethylsilanyl-ethoxy)-pyrrolidin-2-one in 2 ml of trifluoroacetic acid is stirred for 20 hrs. and then concentrated. The residue is dissolved in 5 ml of methanol and treated with 57 mg of sodium bicarbonate in 2 ml of water. The reaction solution is concentrated, the residue is dissolved in 5 ml of water and cooled to 0°C. The crystals obtained are filtered off under suction. There are obtained 117 mg of white crystals. MS (ISP): 584.4 $(M+H)^+$

Example 16

Preparation of (E)-(RS)-3-{5-(2,4-diamino-pyrimidin-5-ylmethyl)-2,3-dimethoxy-phenyl}-1-{1(3,4-dihydroxy-butyl)-1H-phthalazin-2-yl}-propenone.

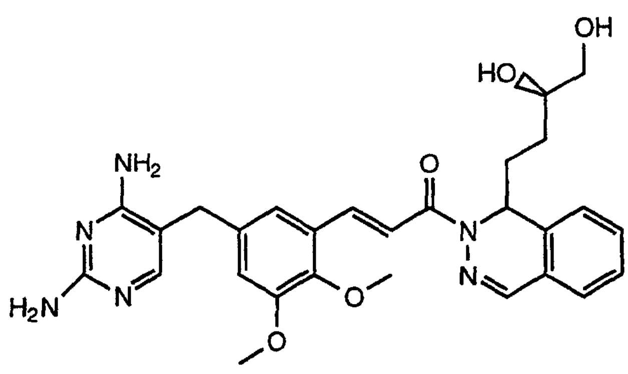

A mixture of 250 mg of (E)-(RS)-3-{5-(2,4-diamino-pyrimidin-5-ylmethyl)-2,3-dimethoxy-phenyl}-1-{1 [2-(2,2-dimethyl-[1,3]-dioxolan-4-yl)-ethyl]-1H-phthalazin-2-yl}-propenone in 4 ml of methanol is treated with 0.65 ml of 2N HCl. The pH value is adjusted to 7-8 after one hour and the mixture is concentrated. The residue is taken up in methanol/dichloromethane, dried over magnesium sulphate and filtered. The filtrate is concentrated and the residue is crystallized from methanol. There are obtained 180 mg of substance. MS (ISP): 533.5 $(M+H)^+$

The educts of formula II which are used can be prepared as follows:

Example 17

17.1. Preparation of 3-iodo-5-methoxy-4-methoxymethoxy-benzaldehyde
14.8 g of potassium carbonate are added a solution of 20 g of 5-iodovanillin in 190 ml of N,N-dimethylformamide. The mixture is cooled to about 10°C and then treated dropwise with 8.1 ml of chloromethyl added thereto within 40 min. The mixture is stirred at room temperature for 30 min., poured on to about 300 ml of ice-water and extracted 5 times with 150 ml of ethyl acetate each time. The organic phases are combined dried, concentrated and the residue is recrystallized from diethyl ether. 15.4 g of 3-iodo-5-methyl-4-methoxymethoxy-benzaldehyde are isolated as a colourless solid.

17.2 Preparation of (E/Z)-2-(3-iodo-5-methoxy-4-methoxymethoxy-benzyl)-3-phenylamino-acrylonitrile
5.0 g of 3-iodo-5-methyl-4-methoxymethoxy-benzaldehyde and 2.73 g of 3-anilinopropionitrile are dissolved in 90 ml of dimethyl sulphoxide and cooled to about 10°C. After the portionwise addition of 2.16 g of potassium tert.-butylate the mixture is stirred at room temperature for a further 2 hrs., then treated with 150 ml of water and 100 ml of diethyl ether. The organic phase is separated, dried and concentrated. After trituration of the residue in diethyl ether 5.65 g of (E/Z)-2-(3-iodo-5-methoxy-4-methoxymethoxy-benzyl)-3-phenylamino-acrylonitrile are obtained as a yellowish solid.

17.3. Preparation of 5-(3-iodo-5-methoxy-4-methoxymethoxy-benzyl)-pyrimidine-2,4-diamine
2.15 g of sodium methylate are dissolved in 190 ml of ethanol. 3.9 g of guanidine hydrochloride are added thereto within 40 min. The mixture is stirred at room temperature for 1 hr. and subsequently treated portionwise with 5.0 g of (E/Z)-2-(3-iodo-5-methoxy-4-methoxymethoxy-benzyl)-3-phenylamino-acrylonitrile. The reaction mixture is held at reflux overnight. The mixture is suction filtered, the insoluble salts are washed with ethanol and the organic phase is dried and concentrated. After recrystallization of the residue from ethanol there are isolated 3.65 g of 5-(3-iodo-5-methoxy-4-methoxymethoxy-benzyl)-pyrimidine-2,4-diamine as a colourless solid, m.p. 180°C, dec.

The preparation of the compounds of formula III (educts of Examples 1-16) is effected as described hereinafter or in analogy thereto.

Example 18

18.1. Preparation of (RS)-1-(1-phenyl-1H-phthalazin-2-yl)-propenone

A solution of 1.11 g of 1-phenyl-1,2-dihydro-phthalazine and 0.67 g of triethylamine in 10 ml of methylene chloride is treated slowly while cooling with ice with a solution of 0.53 g of acryloyl chloride in 10 ml of methylene chloride. Subsequently, the mixture is stirred at room temperature for 20 min., poured on to ice-water and extracted with ethyl acetate. The organic phase is dried, concentrated and chromatographed on 50 g of silica gel; eluent: ethyl acetate/ hexane 3:7 v/v. 1.30 g of (RS)-1-(1-phenyl-1H-phthalazin-2-yl)-propenone are obtained as a colourless oil. MS: 262 ($M^+$).
The following compounds of formula III were prepared in analogy to Example 18.1.:

18.2. (RS)-1-(1-(Pyridin-2-yl)-1H-phthalazin-2-yl)-propenone as a yellow resin. MS : 263 ($M^+$).

18.3. (RS)-1-(1-Furan-2-yl-1H-phthalazin-2-yl)-propenone as a yellow oil. MS : 252 ($M^+$).

18.4. (RS)-1-(1-Butyl-1H-phthalazin-2-yl)-propenone as a yellow oil. MS : 242 ($M^+$).

18.5. (RS)-1-(1-Thiazol-2-yl-1H-phthalazin-2-yl)-propenone as a yellow solid.

18.6. (RS)-2-(2-Acryloyl-1,2-dihydro-phthalazin-1-yl)-N,N-dimethyl-benzenesulphonamide as a greenish, unstable oil. MS (ISP): 370 $(M+H)^+$.

18.7. (RS)-1-[1-(2-Dimethylaminomethyl-phenyl)-1H-phthalazin-2-yl]-propenone as a yellowish unstable oil.

18.8. (RS)-1-[1-(5-Methyl-pyridin-2-yl)-1H-phthalazin-2-yl]-propenone as a brownish solid.

18.9. (RS)-1-[1-(4-Fluoro-phenyl)-1H-phthalazin-2-yl]-propenone as a yellow oil. MS (ISP): 281.1 $(M+H)^+$.

18.10. (RS)-1-[1-(2,4-Difluoro-phenyl)-1H-phthalazin-2-yl]-propenone as a yellowish oil. MS : 298 $(M^+)$.

18.11. (RS)-1-(1-Tridecafluorohexyl-1H-phthalazin-2-yl)-propenone as a yellowish oil. MS (ISP): 505.2 $(M+H)^+$.

18.12. (RS)-1-1-(1-Phenylsulphinylmethyl-1H-phthalazin-2-yl)-propenone as a yellowish oil. MS : 325 $(M+H)^+$.

18.13. (E)-(RS)-1-{1-Methyl-1H-phthalazin-2-yl}-propenone. MS (EI): 200 (M)

18.14. (RS)-1-{1-Ethyl-1H-phthalazin-2-yl}-propenone. MS (EI): 214(M)

18.15. (E)-(RS)-1-{1-Propyl-1H-phthalazin-2-yl}-propenone. MS (EI): 228 (M)

18.16. (E)-(RS)-1-{1-(Isopropyl)-1H-phthalazin-2-yl}-propenone. MS (EI): 228 (M)

18.17. (E)-(RS)-1-{1-(3-Methyl-but-1-yl)-1H-phthalazin-2-yl}-propenone. MS (EI): 256 (M)

18.18. (E)-(RS)-1-{1[2,2-Dimethyl-[1,3]-dioxolan-4-yl]-ethyl}-1H-phthalazin-2-yl}-propenone. MS (ISP): 315.2 $(M+H)^+$

18.19. (E)-(RS)-1-[1-(2-[1,3]-Dioxolan-2-yl-ethyl)-1H-phthalazin-2-yl]-propenone. MS (ISP): 287.3 $(M+H)^+$

18.20. (RS)-1-(1-Cyclohexylmethyl-1H-phthalazin-2-yl)-propenone. MS (ISP): 283.4 $(M+H)^+$

18.21. (RS)- (1-Methylsulfanylmethyl-1H-phthalazin-2-yl)-propenone. MS: 247 $(M^+)$

18.22. (RS)- [1-(4-tert-Butyl-phenyl)-1H-phthalazin-2-yl]-propenenone (yellow oil),

18.23. (RS)- (1-p-Tolyl-1H-phthalazin-2-yl)-propenone (yellow oil),

18.24. (RS)- [1-(4-Ethyl-phenyl)-1H-phthalazin-2-yl]-propenone (yellow oil),

18.25. (RS)- [1-(4-Trifluoromethoxy-phenyl)-1H-phthalazin-2-yl]-propenone (yellow oil),

18.26. (RS)- [1-(3,4-Dimethyl-phenyl)-1H-phthalazin-2-yl]-propenone (yellow oil),

18.27. (RS)- (1-tert-Butyl-1H-phthalazin-2-yl)-propenone MS: 242 $(M^+)$,

18.28. (RS)- [1-(5,6-Dihydro-4H-pyran-2-yl)-1H-phthalazin-2-yl]-propenone MS (ISP): 269.3 $(M+H)^+$,

18.29. (RS)- [1-(2,4,6-Trimethyl-phenyl)-1H-phthalazin-2-yl]-propenone MS (ISP): 305.3 $(M+H)^+$,

18.30 (RS)-1-(1-Biphenyl-4-yl-1H-phthalazin-2-yl)-propenone, m.p. 120°C (diisopropylether). MS (ISP): 339.3 $(M+H)^+$,

18.31 (RS)-1-[1-[4-[1-Ethoxy-ethoxy]-phenyl]-1H-phthalazin-2-yl]-propenone, (yellow oil) MS (ISP): 351.3 $(M+H)^+$,

18.32 (RS)-1-(1-Bicyclo[2.2.1]hept-2endo- and/or 2exo-2-yl-1H-phthalazin-2-yl)-propenone, (yellow oil) MS (ISP): 281.2 $(M+H)^+$

18.33 (RS)-1-[1-(6-Methyl-pyridin-2-yl)-1H-phthalazin-2-yl]-propenone, m.p. 102°C (diisopropylether). MS (ISP): 278.2 $(M+H)^+$,

18.34 (RS)-1-[1-(4-Dimethylamino-phenyl)-1H-phthalazin-2-yl]-propenone, m.p. 118-122°C (ethyl acetate/hexane). MS (ISP): 306.3 $(M+H)^+$.

18.35 (RS)-1-{1-[4-(2-Ethoxy-ethoxy)-phenyl]-1H-phthalazin-2-yl}-propenone, b.p. 205°C/0.06 mbar MS (ISP):

351.3 $(M+H)^+$.

18.36 (RS)-1-(1-m-Tolyl-1H-phthalazin-2-yl)-propenone, b.p. 170°C/0.1 mbar MS (EI): 276 (M).

18.37 (RS)-1-[1-(3,5-Dimethyl-phenyl)-1H-phthalazin-2-yl]-propenone m.p. 110°C (hexane). MS (ISP): 291.2 $(M+H)^+$.

18.38 (RS)-1-[1-(4-Pyrrol-1-yl-phenyl)-1H-phthalazin-2-yl]-propenone, (red oil), MS (ISP): 328.2 $(M+H)^+$.

18.39 (RS)-1-[1-(2,3-Dihydro-benzo[1,4]dioxin-6-yl)-1H-phthalazin-2-yl]-propenone, (yellow oil),

18.40 (RS)-1-(1-Cyclopropyl-1H-phthalazin-2-yl)-propenone, b.p. 120-125°C/0.08 mbar MS (EI): 226 (M),

18.41 (RS)-1-[1-(4-Methylsulfanyl-phenyl)-1H-phthalazin-2-yl]-propenone, m.p. 95-98°C (ethyl acetate/hexane). MS (EI): 308 (M),

18.42 (RS)-1-[1-(6-Methyl-pyridin-3-yl)-1H-phthalazin-2-yl]-propenone, m.p. 76-79°C (ethyl acetate/hexane). MS (ISP): 278.2 $(M+H)^+$,

18.43 (RS)-1-(1-[1,3]Dithian-2-yl-1H-phthalazin-2-yl)-propenone, m.p. 140-143°C (ethyl acetate/hexane). MS (ISP): 305.2 $(M+H)^+$.

18.44 (RS)-1-[1-(6-Dimethylamino-pyridin-3-yl)-1H-phthalazin-2-yl]-propenone, m.p. 125-127°C (ethyl acetate/hexane), MS (ISP): 307.2 $(M+H)^+$.

18.45 (RS)-1-(1-Pyridin-3-yl-1H-phthalazin-2-yl)-propenone, MS (ISP): 264.3 $(M+H)^+$.

18.46 (RS)-1-(1-Cyclobutyl-1H-phthalazin-2-yl)-propenone, b.p. 120°C/0.05 mbar MS (ISP): 241.4 $(M+H)^+$.

18.47 (RS)-1-(1-Cyclopentyl-1H-phthalazin-2-yl)-propenone, b.p. 150°C/0.12 mbar MS (EI): 254 (M).

18.48 (RS)-1-[1-(4-Trifluoromethyl-phenyl)-1H-phthalazin-2-yl]-propenone, m.p. 90-91°C (hexane). MS (ISP): 331.2 $(M+H)^+$.

18.49 (RS)-1-[1-(5-Chloro-pyridin-2-yl)-1H-phthalazin-2-yl]-propenone, m.p. 131-132°C (ethyl acetate/hexane) MS (ISP): 298.2 $(M+H)^+$.

18.50 (RS)-1-(1-Cyclohexyl-1H-phthalazin-2-yl)-propenone, b.p. 135°C/0.08 mbar MS (EI): 268 (M).

18.51 (RS)-1-[1-(4-Methoxy-phenyl)-1H-phthalazin-2-yl]-propenone, b.p. 165°C/0.1 mbar MS (ISP): 293.3 $(M+H)^+$.

18.52 (RS)-1-[1-(3,4-Dimethoxy-phenyl)-1H-phthalazin-2-yl]-propenone, b.p. 124-125°C (ethyl acetate/hexane) MS (ISP): 323.3 $(M+H)^+$.

18.53 (RS)-1-(1-Benzo[1,3]dioxol-5-yl-1H-phthalazin-2-yl)-propenone, m.p. 98-101°C (ethyl acetate/hexane) MS (EI): 306 (M).

18.54 (RS)-4-(2-Acryloyl-1,2-dihydro-phthalazin-1-yl)-benzonitrile,

18.55 (RS)-4-(2-Acryloyl-1,2-dihydro-phthalazin-1-yl)-benzoic acid tert-butyl ester,

18.56 (RS)-1-(1-Thiophen-2-yl-1H-phthalazin-2-yl)-propenone, (orange oil) MS (EI): 268 (M).

18.57 (RS)-1-[1-(5-Butyl-thiophen-2-yl)-1H-phthalazin-2-yl]-propenone, (yellow oil) MS (EI): 324 (M).

18.58 (RS)- 1-{1-[4-(1-Hydroxy-ethyl)-phenyl]-1H-phthalazin-2-yl}-propenone,

18.59 (RS)-1-(1-Benzenesulfonylmethyl-1H-phthalazin-2-yl)-propenone, m.p. 113-116°C (ethyl acetate/hexane) MS (ISP): 341.1 $(M+H)^+$.

18.60 (RS)-4-(2-Acryloyl-1,2-dihydro-phthalazin-1-yl)-N,N-dimethyl-benzenesulfonamide, MS (ISP): 370.2 $(M+H)^+$.

18.61 (RS)-1-[1-(4-Methoxymethyl-phenyl)-1H-phthalazin-2-yl]-propenone, b.p. 200°C/0.11 mbar MS (ISP): 307.2 $(M+H)^+$.

18.62 (RS)-1-(1-[4-{(Diisopropylamino)-methyl]-phenyl}-1H-phthalazin-2-yl)-propenone, m.p. 99-101°C (hexane). MS (ISP): 376.4 $(M+H)^+$.

18.63 (RS)-1-[1-(4-Chloro-phenyl)-1H-phthalazin-2-yl]-propenone, m.p. 71-72°C (ethyl acetate/hexane). MS (ISP): 297.2 $(M+H)^+$.

18.64 (RS)-1-[1-(6-Methoxy-pyridin-3-yl)-1H-phthalazin-2-yl]-propenone, m.p. 128-130°C (ethyl acetate/hexane). MS (ISP): 294.3 $(M+H)^+$.

18.65 (RS)-1-{1-[4-(4-Methyl-piperazin-1-ylmethyl)-phenyl]-1H-phthalazin-2-yl}-propenone, (yellow oil) MS (ISP): 375.4 $(M+H)^+$.

18.66 (RS)-1-[1-(4-Morpholin-4-ylmethyl-phenyl)-1H-phthalazin-2-yl]-propenone, (yellow oil). MS (ISP): 362.2 $(M+H)^+$.

18.67 (RS)-1-(1-Adamantan-1-yl-1H-phthalazin-2-yl)-propenone, m.p. 157-158°C (hexane). MS (ISP): 321.3 $(M+H)^+$.

18.68 RS)-1-{1-[6-(1-Hydroxy-1-methyl-ethyl)-pyridin-3-yl]-1H-phthalazin-2-yl}-propenone,

18.69 (RS)-1-[1-(6-Morpholin-4-yl-pyridin-3-yl)-1H-phthalazin-2-yl]-propenone, (yellow oil) MS (ISP): 349.4 $(M+H)^+$.

18.70 (RS)-5-(2-Acryloyl-1,2-dihydro-phthalazin-1-yl)-pyridin-2-carbonic acid tert-butylamide, MS (EI): 362 (M).

The (+)-enantiomere compounds and (-)-enantiomere compounds respectively, can be prepared from the racemic [1H-Phthalazin-2-yl]-propenone by HPLC (e.g. ChiralCell OD). Thus are obtained:

18.71. S-(+)-(1-Phenyl-1H-phthalazin-2-yl)-propenone; $[\alpha]_D$ = +724° (c=1 ; methanol).

18.72. R-(-)-(1-Phenyl-1H-phthalazin-2-yl)-propenone; $[\alpha]_D$ = -720° (c=1 ; methanol).

18.73. R-(+)-(1-Furan-2-yl-1H-phthalazin-2-yl)-propenone; $[\alpha]_D$ = +638° (c=1 ; methanol).

18.74. S-(-)-(1-Furan-2-yl-1H-phthalazin-2-yl)-propenone; $[\alpha]_D$ = -622° (c=1 ; methanol).

18.75. S-(+)-(1-Propyl-1H-phthalazin-2-yl)-propenone ; $[\alpha]_D$ = +861° (c=1 ; methanol).

18.76. R-(-)-(1-Propyl-1H-phthalazin-2-yl)-propenone ; $[\alpha]_D$ = -902° (c=1 ; methanol).

18.77. S-(+)-(1-Methyl-1H-phthalazin-2-yl)-propenone ; $[\alpha]_D$ = +874° (c=1 ; methanol).

18.78. R-(-)-(1-Methyl-1H-phthalazin-2-yl)-propenone ; $[\alpha]_D$ = -903° (c=1 ; methanol).

18.79 (E)-(R)-1-[1-(6-Methyl-pyridin-3-yl)-1H-phthalazin-2-yl]-propenone, b.p. 145°C/0.08 mbar, $[\alpha]_D$= +571° (c=1, methanol). MS (ISP): 278.1 $(M+H)^+$.

18.80 (E)-(S)-1-[1-(6-Methyl-pyridin-3-yl)-1H-phthalazin-2-yl]-propenone, b.p. 150°C/0.1 mbar, $[\alpha]_D$= -536° (c=0.8, methanol). MS (ISP): 278.2 $(M+H)^+$.

18.81 (R)-1-(1-Cyclopropyl-1H-phthalazin-2-yl)-propenone, b.p. 120°C/0.1 mbar, $[\alpha]_D$= +960° (c=1, methanol). MS (EI): 226 (M).

18.82 (S)-1-(1-Cyclopropyl-1H-phthalazin-2-yl)-propenone, b.p. 120°C/0.1 mbar , $[\alpha]_D$ = -958° (c=1, methanol). MS (EI): 226 (M).

18.83 (R)-1-[1-(4-Methylsulfanyl-phenyl)-1H-phthalazin-2-yl]-propenone, m.p. 98-104°C, $[\alpha]_D$ = +623° (c=1, chloroform). MS (ISP): 309.1 $(M+H)^+$.

18.84 (S)-1-[1-(4-Methylsulfanyl-phenyl)-1H-phthalazin-2-yl]-propenone, m.p. 98-104°C, $[\alpha]_D$= -635° (c=1, chloroform). MS (EI): 308 (M).

The compounds of formula III in which $R^3$ is cyclopentanone are prepared as follows:

Example 19

Preparation[of (RS)-2-(2-acryloyl-1,2-dihydro-phthalazin-1-yl)-cyclopentanone

A solution of 317 mg of acryloyl chloride in 1.5 ml of methylene chloride is slowly added dropwise at 0°C to a solution of 390 mg of phthalazine and 536 mg of 1-morpholinocyclopentene in 10 ml of methylene chloride. The mixture is stirred at 0°C for 2 hrs., then concentrated, treated with ice-water and made basic by the addition of sodium carbonate. The mixture is extracted 3 times with ethyl acetate. The organic phases are combined, dried, filtered, the filtrate is concentrated and the residue is chromatographed on 65 g of silica gel; eluent: cyclohexane/ethyl acetate 4: 1. 136 mg of (RS)-2-(2-acryloyl-1,2-dihydro-phthalazin-1-yl)-cyclopentanone (mixture of diastereomers) are isolated as a yellowish oil. MS: 268 $(M^+)$.

The compounds of formula III in which $R^3$ is cyclohexanone are prepared as follows:

Example 20

Preparation of (RS)-2-(2-acryloyl-1,2-dihydro-phthalazin-1-yl)-cyclohexanone

A solution of 0.53 ml of acryloyl chloride in 3 ml of methylene chloride is slowly added dropwise at 0°C to a solution of 0.78 g of phthalazine and 1.2 ml of 1-trimethylsiloxy-cyclohexene. After the addition the mixture is stirred at 0°C for a further 30 min., then concentrated, treated with ice-water and made basic by the addition of sodium carbonate. The mixture is extracted 3 times with 100 ml of ethyl acetate each time. The organic phases are combined, dried over magnesium sulphate, filtered and the filtrate is concentrated. 1.63 g of (RS)-2-(2-acryloyl-1,2-dihydrophthalazin-1-yl)-cyclohexanone (mixture of diastereomers) are isolated as a brownish oil. MS: 282 $(M^+)$.

The following compounds of formula III are prepared in analogy to Example 20.1.

20.2. (RS)-1-[1-(2-Oxo-propyl)-1H-phthalazin-2-yl]-propenone as a brownish oil. MS : 242 $(M^+)$.

20.3. Methyl (RS)-2-(2-acryloyl-1,2-dihydro-phthalazin-1-yl)-2-methyl-propionate as a brownish oil. MS : 286 $(M^+)$.

20.4. (RS)-1-[1-(1-Methyl-2-oxo-2-phenyl-ethyl)-1H-phthalazin-2-yl]-propenone (mixture of diastereomers) as a brownish oil. MS: 318 $(M^+)$.

20.5. (RS)-1-[1-(2-Oxo-2-phenyl-ethyl)-1H-phthalazin-2-yl]-propenone as a yellow oil. MS : 304 $(M^+)$.

20.6. (RS)-3-(2-Acryloyl-1,2-dihydro-phthalazin-1-yl)-tetrahydro-pyran-2-one (mixture of diastereomers) as a yellow foam. MS : 284 $(M^+)$.

20.7. (RS)-1-[1-(2-Furan-2-yl-2-oxo-ethyl)-1H-phthalazin-2-yl]-propenone as a brownish oil. MS: 294 $(M^+)$.

The compounds of formula III in which $R^3$ is hydrogen are prepared as follows:

Example 21

Preparation (sic) of 1-(1H-phathalazin-2-yl)-propenone

A solution of 1 g of phthalazine in 10 ml of methanol is treated at -78°C with 290 mg of sodium borohydride and stirred for a further 10 min. A solution of 0.75 g of acryloyl chloride in 5 ml of diethyl ether is added dropwise at -78°C during 45 min. and the pale yellow suspension is stirred at -78°C for a further 1 hr. The mixture is poured on to 50 ml of ice-water, treated with 50 ml of a 10% aqueous sodium carbonate solution and extracted with 3 X 50 ml of methylene chloride. The organic phases are combined and washed with 100 ml of water and 50 ml of a saturated aqueous sodium chloride solution. The organic phase is dried over magnesium sulphate, filtered, the filtrate is concentrated and the residue is chromatographed on 80 g of silica gel; eluent: ethyl acetate. 1.22 g of 1-(1H-phthalazin-2-yl)-propenone are isolated as a pale yellow oil. MS: 186 ($M^+$).

Compounds of formula III in which $R^3$ carries a hydroxyalkyl group are prepared as follows:

Example 22

22.1. Preparation of (E)-(RS)-1-{1-(4-hydroxymethyl-phenyl)-1H-phthalazin-2-yl}-propenone.

A solution of 730 mg of E)-(RS)-1-{1-(4-tetrahydro-pyran-2-yloxymethyl)-phenyl]-1H-phthalazin-2-yl}-propenone in 20 ml of methanol is treated with a spatula tip of para-toluenesulphonic acid. After two hours the reaction mixture is concentrated and the residue is chromatographed on silica gel. 320 mg of $CH_2Cl_2$/MeOH/ $NH_4OH$: 95/5/0.5) are obtained as a colourless oil. MS (EI): 292 (M).

The educt (E)-(RS)-1-{1-(4-tetrahydro-pyran-2-yloxymethyl)-phenyl]-1H-phthalazin-2-yl}-propenone, MS (ISP): 377.3 $(M+H)^+$, used in Reaction 22 is prepared by reacting the corresponding 4-tetrahydro-pyran-2-yloxymethyl)-phenyl bromide with phthalazine in the presence of butyllithium.

The following compounds of formula III in which $R^3$ carries a hydroxyalkyl group were prepared in analogy to Example 22.1.

22.2. (E)-(RS)-1-{1-(2-Hydroxymethyl-phenyl)-1H-phthalazin-2-yl}-propenone. MS (ISP): 293.2 $(M+H)^+$

The educt (E)-(RS)-1-{1-(2-tetrahydro-pyran-2-yloxymethyl)-phenyl]-1H-phthalazin-2-yl}-propenone, MS (ISP): 377.4 $(M+H)^+$, is [prepared] from the corresponding 2-tetrahydro-pyran-2-yloxymethyl-phenyl bromide by reaction with butyllithium.

22.3. (E)-(RS)-1-{1-(3-Hydroxymethyl-phenyl)-1H-phthalazin-2-yl}-propenone. MS (ISP): 293.2 $(M+H)^+$

The educt (E)-(RS)-1-{1-(3-tetrahydro-pyran-2-yloxymethyl)-phenyl]-1H-phthalazin-2-yl}-propenone, MS (ISP): 377.2 $(M+H)^+$, is prepared from the corresponding 1-(3-tetrahydro-pyran-2-yloxymethyl)-phenyl] bromide by reaction with butyllithium.

22.4. (E)-(RS)-1-{1-(4-(2-Hydroxyethyl)-phenyl)-1H-phthalazin-2-yl}-propenone. MS(ISP): 307.3 $(M+H)^+$

The educt (E)-(RS)-1-{1-[4-(2-tetrahydro-pyran-2-yloxy)-ethyl]-phenyl}-1H-phthalazin-2-yl}-propenone MS (EI): 390 (M), is prepared from the corresponding 1-[4-(2-tetrahydro-pyran-2-yloxy) ethyl]-phenyl} bromide by reaction with butyllithium.

22.5. (E)-(RS)-1-{1-(4-Hydroxybutyl)-1H-phthalazin-2-yl}-propenone, colourless oil, MS (EI): 258 (M).

The educt (E)-(RS)-1-{1-[4- [2-tetrahydro-pyran-2-yloxy]-butyl]-1H-phthalazin-2-yl}-propenone, MS (ISP): 343.3 $(M+H)^+$, is prepared from the corresponding Grignard reagent (from 1-[4-( 2-tetrahydro-pyran-2-yloxy)-ethyl]-phenyl bromide.

Compounds of formula III in which $R^3$ carries a hydroxy group are prepared as follows:

Example 23

23.1. Preparation of (E)-(RS)-1-{1-(4-hydroxy-phenyl)-1H-phthalazin-2-yl}-propenone.

A solution [of] 740 mg of 1-{1-[4-(tert-butyl-dimethylsilanyloxy)-phenyl]-1H-phthalazin-2-yl}-propenone in 20

ml of tetrahydrofuran is treated at about 0°C with 1 ml of 1M tetrabutylammonium fluoride solution. After two hours the solvent is evaporated and the residue, dissolved in ethyl acetate, is washed with water and saturated sodium chloride solution. The organic phase is dried over magnesium sulphate, filtered, the filtrate is concentrated and the residue is chromatographed on silica gel (eluent: ethyl acetate/hexane: 1/1). The pure fractions are combined, concentrated and triturated in hexane. 404 mg of (E)-(RS)-1-{1-(4-hydroxy-phenyl)-1H-phthalazin-2-yl}-propenone are obtained as a white solid. MS (ISP): 279.2 $(M+H)^+$

The educt 1-{1-[4-(tert-butyl-dimethylsilanyloxy)-phenyl]-1H-phthalazin-2-yl}-propenone, MS (ISP): 393.4 $(M+H)^+$, is prepared from the corresponding 4-tert-butyl-dimethylsilanyloxy)-phenyl] bromide by reaction with butyllithium.

The following compound was prepared in analogy to Example 23.1.:

23.2. bb) (E)-(RS)-1-{1-(3-Hydroxy-phenyl)-1H-phthalazin-2-yl}-propenone. MS (ISP): 279.2 $(M+H)^+$

The educt 1-{1-[3-(tert-butyl-dimethylsilanyloxy)-phenyl]-1H-phthalazin-2-yl}-propenone, MS (EI): 392 (M), was prepared from the corresponding 3-(tert-butyl-dimethylsilanyloxy)-phenyl] bromide by reaction with butyllithium

Compounds of formula III in which $R^3$ carries a carbamoyl group are prepared as follows:

Example 24

24.1 Preparation of (E)-(RS)-carbamic acid 4-(2-acryloyl-1,2-dihydrophthalazin-1-yl)-benzyl ester.

A solution of 292 mg of E)-(RS)-1-{1-(4-hydroxymethyl-phenyl)-1H-phthalazin-2-yl}-propenone in 20 ml of dichloromethane is treated with 282 mg of disuccinyl carbonate and 61 mg of 4-dimethylaminopyridine. After 2 hrs. the solution is diluted with 20 ml of tetrahydrofuran and treated with 0.5 ml of triethylamine sic and 2 ml of saturated sodium chloride solution. After 12 hrs. the solvents are evaporated. The residue is dissolved in dichloromethane and washed with water. The organic phase is dried over magnesium sulphate, filtered, the filtrate is concentrated and the residue is chromatographed on silica gel (eluent: ethyl acetate/hexane: 2/1). The pure fractions are combined, concentrated. 210 mg of white product are obtained. MS (ISP): 336.2 $(M+H)^+$ 353.3 $(M+NH_4)^+$

The following compounds were prepared in analogy to Example 23.1.:

24.2. (E)-(RS)-Morpholine-1-carbamic acid 4-[4-(2-acryloyl-1,2-dihydro-phthalazin-1-yl)-phenyl]-butyl ester. MS (ISP): 372.3 $(M+H)^+$

24.3. (E)-(RS)-Carbamic acid 4-(2-acryloyl-1,2-dihydrophthalazin-1-yl)-butyl ester MS (ISP): 302.2 $(M+H)^+$

24.4. (E)-(RS)-Carbamic acid 4-(2-acryloyl-1,2-dihydrophthalazin-1-yl)-propyl ester MS (ISP): 288.3 $(M+H)^+$

Compounds of formula III in which the $R^3$ carries an aldehyde group are prepared as follows:

Example 25

Preparation of (E)-(RS)-3-(2-acryloyl-1,2-dihydro-phthalazin-1-yl)-propion-aldehyde

A solution of 500 mg of (E)-(RS)-1-[1-(2-[1,3]-dioxolan-2-yl-ethyl)-1H-phthalazin-2-yl]-propenone in 15 ml of ethanol is treated with 3 ml of 2N HCl. After twenty hours at 60C the reaction mixture is concentrated and the residue is diluted in ethyl acetate, washed with saturated sodium bicarbonate and saturated sodium chloride solution then dried over magnesium sulphate filtered, the filtrate is concentrated and the residue is chromatographed on silica gel (eluent $CH_2Cl_2$/ether 95:). 50 mg of material [as a] colourless oil are obtained. MS (EI): 242 (M)

Compounds of formula III in which the $R^3$ carries an alkylhydroxy group can be prepared from the corresponding aldehyde as follows:

Example 26

Preparation [of] (E)-(RS)-1-[1-(3-hydroxy-propyl))-1H-phthalazin-2-yl]-propenone

A solution of 220 mg in 3 ml of ethanol is treated at about 0°C with 13 mg of sodium borohydride. After one hour the solution is concentrated, the residue is diluted with ethyl acetate, washed with saturated sodium bicarbonate and saturated sodium chloride solution then dried over magnesium sulphate, filtered, the filtrate is concentrated and

the residue is chromatographed on silica gel (eluent hexane/ether 6:4). 176 mg of material are obtained. MS (EI): 244 (M)

The dihydro-phthalazines of formula IV used for the preparation of compounds III are generally relatively unstable, oxidation-sensitive compounds, which are often reacted directly in the crude state with acryloyl halides. They can be prepared as described hereinafter or in analogy thereto:

Example 27

27.1 Preparation of 1-phenyl-1,2-dihydro-phthalazine

A solution of 4 g of phthalazine in 50 ml of tetrahydrofuran is added dropwise at room temperature to a solution of phenyl-magnesium bromide (prepared from 6 g of bromobenzene and 1 g of magnesium in 30 ml of tetrahydrofuran). The reaction mixture is held at reflux for 6 hrs. After cooling the mixture is treated with a saturated aqueous solution of ammonium chloride and extracted 3 times with diethyl ether. The organic phases are combined, dried and subsequently triturated with pentane. 2.32 g of not quite pure 1-phenyl-1,2-dihydro-phthalazine are isolated as a yellowish solid, m.p. 79°C.

The following compounds of formula IV are prepared in analogy to Example 27.1.

27.2. 1-Pyridin-2-yl-1,2-dihydro-phthalazine as a brownish oil MS : 208 ($M^+$).

27.3. 1-Thiazol-2-yl-1,2-dihydro-phthalazine as a brownish oil.

27.4. 1-Butyl-1,2-dihydro-phthalazine as a brownish oil.

27.5 1-(5-Methyl-pyridin-2-ylI-1,2-dihydro-phthalazine as a dark impure oil.

27.6. (RS)1-(2,4-Difluoro-phenyl)-1,2-dihydro-phthalazine as a reddish oil. MS: 245 $(M + H)^+$.

27.7. (RS)-1-Cyclobutyl-1,2-dihydro-phthalazine m.p. 64-68°C (hexane). MS (ISP): 187.3 $(M+H)^+$.

27.8. (RS)-1-Cyclopentyl-1,2-dihydro-phthalazine, brown solid

27.9. (RS)-1-Cyclohexyl-1,2-dihydro-phthalazine, m.p. 122-124°C (hexane), MS (ISP): 215.4 $(M+H)^+$.

Example 28

28.1. Preparation of 1-furan-2-yl-1,2-dihydrophthalazine

22.5 ml of an about 1.6M solution of butyllithium are slowly added dropwise at -78°C to a solution of 2.46 g of furan in 30 ml of tetrahydrofuran. The mixture is stirred at -78°C for 20 min., then at -20°C for 2 hrs. The solution is again cooled to -78°C and treated slowly with a solution of 4.68 g of phthalazine in 36 ml of tetrahydrofuran. The mixture is stirred at -78°C for 2 hrs., then poured on to about 500 ml of ice-water and extracted 4 times with 150 ml of ethyl acetate each time. The organic phases are dried, concentrated and the residue is chromatographed immediately on 120 g of silica gel; eluent: hexane/ethyl acetate 3:7. 4.08 g of dark, impure 1-furan-2-yl-1,2-dihydrophthalazine are isolated.

The following compounds of formula IV are prepared in analogy to Example 28.1.:

28.2. 2-(1,2-Dihydro-phthalazin-1-yl)-N,N-dimethyl-benzenesulphonamide as an impure, dark oil, MS: 315 ($M^+$).

28.3. [2-(1,2-Dihydro-phthalazin-1-yl)-benzyl]-dimethyl-amine as a dark, very unstable oil.

28.4. (RS)- 1-Methylsulfanylmethyl-1H-phthalazine as an impure, yellow oil,

28.5. (RS)- 1-(4-tert-Butyl-phenyl)-1H-phthalazine as an impure, yellow oil,

28.6. (RS)- 1-p-Tolyl-1H-phthalazine as an impure, yellow oil,

28.7. (RS)- 1-(4-Ethyl-phenyl)-1H-phthalazine as an impure, yellow oil,

28.8. (RS)- 1-(4-Trifluoromethoxy-phenyl)-1H-phthalazine as an impure, yellow oil,

28.9. (RS)- 1-(3,4-Dimethyl-phenyl)-1H-phthalazine as an impure, yellow oil,

28.10. (RS)- 1-tert-Butyl-1H-phthalazine as an impure, yellow oil,

28.11. (RS)- 1-(5,6-Dihydro-4H-pyran-2-yl)-1H-phthalazine MS (ISP): 213.3 $(M+H)^+$.

28.12. (RS)- 1-(2,4,6-Trimethyl-phenyl)-1H-phthalazine as an impure, yellow oil,

28.13 (RS)-1-Biphenyl-4-yl-1,2-dihydro-phthalazine, yellow oil,

28.14 (RS)-1-[1-[4-[1-Ethoxy-ethoxy]-phenyl]-1H-phthalazine, yellow oil,

28.15 (RS)-1-Bicyclo[2.2.1]hept-2endo- and/or 2exo 2-yl-1,2-dihydrophthalazine, brown oil,

28.16 (RS)-1-(6-Methyl-pyridin-2-yl)-1,2-dihydro-phthalazine, yellow oil,

28.17 (RS)-[4-(1,2-Dihydro-phthalazin-1-yl)-phenyl]-dimethyl-amine, yellow oil,

28.18 (RS)-1-[4-(2-Ethoxy-ethoxy)-phenyl]-1,2-dihydro-phthalazine, m.p. 129-131°C (acetylacetate/hexane). MS (EI): 296 (M).

28.19 (RS)-1-m-Tolyl-1,2-dihydro-phthalazine, brown oil

28.20 (RS)-1-(3,5-Dimethyl-phenyl)-1,2-dihydro-phthalazine, m.p. 102-107°C (hexane). MS (ISP): 237.3 $(M+H)^+$.

28.21 (RS)-1-(4-Pyrrol-1-yl-phenyl)-1,2-dihydro-phthalazine, m.p. 129-130°C (toluol/hexane). MS (ISP): 274.3 $(M+H)^+$.

28.22 (RS)-1-(2,3-Dihydro-benzo[1,4]dioxin-6-yl)-1,2-dihydro-phthalazine, yellow oil,

28.23 (RS)-1-Cyclopropyl-1,2-dihydro-phthalazine, yellow oil,

28.24 (RS)-1-(4-Methylsulfanyl-phenyl)-1,2-dihydro-phthalazine, m.p. 120-123°C (acetylacetate/hexane), MS (ISP): 255.2 $(M+H)^+$.

28.25 (RS)-1-(6-Methyl-pyridin-3-yl)-1,2-dihydro-phthalazine, yellow oil.

28.26 (RS)-1-[1,3]Dithian-2-yl-1,2-dihydro-phthalazine, m.p. 134-136°C (diisopropylether). MS (ISP): 251.1 $(M+H)^+$.

28.27 (RS)-[5-(1,2-Dihydro-phthalazin-1-yl)-pyridin-2-yl]-dimethyl-amine, m.p. 124-127°C (acetylacetate/hexane). MS (ISP): 253.2 $(M+H)^+$.

28.28 (RS)-1-Pyridin-3-yl-1,2-dihydro-phthalazine,

28.29 (RS)-1-(4-Trifluoromethyl-phenyl)-1,2-dihydro-phthalazine,

28.30 (RS)-1-(5-Chloro-pyridin-2-yl)-1,2-dihydro-phthalazine,

28.31 (RS)-1-(4-Methoxy-phenyl)-1,2-dihydro-phthalazine, m.p. 121-125°C (acetylacetate/hexane). MS (EI): 238 (M).

28.32 (RS)-1-(3,4-Dimethoxy-phenyl)-1,2-dihydro-phthalazine, brown oil,

28.33 (RS)-1-Benzo[1,3]dioxol-5-yl-1,2-dihydro-phthalazine, m.p. 102-104°C (acetylacetate/hexane). MS (ISP): 253.2 $(M+H)^+$.

28.34 (RS)-4-(1,2-Dihydro-phthalazin-1-yl)-benzonitrile, red oil,

28.35 (RS)-4-(1,2-Dihydro-phthalazin-1-yl)-benzoic acid tert-butyl ester,

28.36 (RS)-1-Thiophen-2-yl-1,2-dihydro-phthalazine,

28.37 1-[4-(1,2-Dihydro-phthalazin-1-yl)-phenyl]-ethanol, m.p. 133-134°C (acetylacetate/hexane). MS (ISP): 253.2 $(M+H)^+$.

28.38 (RS)-1-Benzenesulfonylmethyl-1,2-dihydro-phthalazine, orange oil.

28.39 (RS)-4-(1,2-Dihydro-phthalazin-1-yl)-N,N-dimethyl-benzene-sulfonamide,

28.40 (RS)-1-(4-Methoxymethyl-phenyl)-1,2-dihydro-phthalazine, brown oil,

28.41 (RS)-[4-(1,2-Dihydro-phthalazin-1-yl)-benzyl]-diisopropyl-amine, brown oil,

28.42 (RS)-1-(4-Chloro-phenyl)-1,2-dihydro-phthalazine, m.p. 97-98°C (hexane), MS (EI): 242 (M).

28.43 (RS)-1-(6-Methoxy-pyridin-3-yl)-1,2-dihydro-phthalazine,

28.44 (RS)-1-[4-(4-Methyl-piperazin-1-ylmethyl)-phenyl]-1,2-dihydrophthalazine, yellow oil,

28.45 (RS)-1-(4-Morpholin-4-ylmethyl-phenyl)-1,2-dihydro-phthalazine, brown oil,

28.46 (RS)-1-Adamantan-1-yl-1,2-dihydro-phthalazine, m.p. 170-172°C, dec. (hexane). MS (ISP): 267.4 $(M+H)^+$.

28.47 (RS)-1-(6-Morpholin-4-yl-pyridin-3-yl)-1,2-dihydro-phthalazine, m.p. 167°C (acetylacetate). MS (ISP): 295.3 $(M+H)^+$.

Example 29

Preparation of (RS)-1-(4-fluoro-phenyl)-1,2-dihydro-phthalazine

5.53 ml of a 1.6 M solution of butyllithium in hexane are added dropwise at -78°C within 20 min. to a solution of 1.48 g of 1-bromo-4-fluoro-benzene in 5 ml of tetrahydrofuran. The resulting white suspension is stirred for a further hour. A solution of 1 g of phthalazine in 5 ml of tetrahydrofuran is added dropwise at -78°C within 10 min. The reaction mixture is left to warm to room temperature, treated with 50 ml of water and extracted 3 times with 50 ml of dichloromethane each time. The organic phases are combined washed with 50 ml of water and 50 ml of a saturated sodium chloride solution, dried over magnesium sulphate, filtered, the filtrate is concentrated and the residue is chromatographed on 100 g of silica gel with the eluent hexane/ethyl acetate 1:1. 1.49 g (86%) of (RS)-1-(4-fluoro-phenyl)-1,2-dihydro-phthalazine are obtained as a yellow oil. MS: 227 $(M+H)^+$.

Example 30

Preparation of (RS)-1-benzenesulphinylmethyl-1,2-dihydro-phthalazine

A solution of 1 g of methyl phenyl sulphoxide in 14 ml of tetrahydrofuran is added dropwise at 78°C within 10 min. to 3.93 ml of 2M solution of lithium diisopropylamide in tetrahydrofuran/heptane/ethylbenzene in 20 ml of tetrahydrofuran. The reaction mixture is stirred for a further 30 min. A solution of 0.928 g of phthalazine in 14 ml of tetrahydrofuran is added dropwise within 10 min. The mixture is stirred at -78°C for 1 hr., treated with 100 ml of water and extracted 3 times with 50 ml of chloroform. The organic phases are combined, dried over magnesium sulphate, filtered and the filtrate is concentrated. 1.9 g of crude (RS)-1-benzenesulphinylmethyl-1,2-dihydro-phthalazine are isolated as a yellowish oil, MS(ISP): 271.2 $(M+H)^+$.

Example 31

Preparation of (RS)-1-(6-Methyl-pyridin-3-yl)-1,2-dihydro-phthalazine

15.3 ml of a 1.6 M solution of butyllithium in hexane are added dropwise at -78°C within 20 min. to a solution of 5.68 g 2,5-dibromo-pyridine in 200 ml diethylether. Under stirring at -78°C the following solutions are added dropwise:

a solution of 2.6 g phthalazine in 100 ml tetrahydrofuran within 15 min., 16.9 ml of a 1.6 M solution of butyllithium in hexane within 5 min. and 2.24 ml methyliodide.

The reaction mixture is stirred for a further 30 min and treated with 100 ml water and 100 ml of a saturated sodium chloride solution. The organic phases are combined, dried over sodium sulphate and concentrated. 5.4 g (RS)-1-(6-methyl-pyridin-3-yl)-1,2-dihydro-phthalazine are obtained as yellow oil.

The following compounds are prepared in analogy to the preparation of (RS)-1-(6-Methyl-pyridin-3-yl)-1,2-dihydro-phthalazine, as described above.

31.1 (RS)-2-[5-(1,2-Dihydro-phthalazin-1-yl)-pyridin-2-yl]-propan-2-ol, brown solid.

31.2. (RS)-5-(1,2-Dihydro-phthalazin-1-yl)-pyridin-2-carbonic acid tert-butylamide, brown solid.

Example 32

Preparation of (E)-(RS)-3-[5-(2,4-Diamino-pyrimidin-5-ylmethyl)-2,3-dimethoxy-phenyl]-1-[1-(6-methylsulfanyl-pyridin-3-yl)-1H-phthalazin-2-yl]-propenone

8. 93 ml of a 1.6 M solution of butyllithium in hexane are added dropwise at -78°C within 20 min. to a solution of 2. 86 g 5-bromo-2-methyl-sulfanyl-pyridine in 50 ml diethylether. Under stirring at -78°C are a solution of 1. 56 g phthalazine in 50 ml tetrahydrofuran is added dropwise within 15 min. (solution A)

15.75 ml of a 1.6 M solution of butyllithium in hexane are added dropwise at -78°C within 10 min. to a solution of 4.3 g (E)-3-[5-(2,4-diamino-pyrimidin-5-ylmethyl)-2,3-dimethoxy-phenyl]-acrylacid ethyl ester in 430 ml tetrahydrofuran. Solution A is added. The reaction mixture is stirred for a further 3 h. The termperature raises up to -10°C. 150 ml water and 100 ml of a saturated sodium chloride solution are added. The organic phases are combined, dried over sodium sulphate and concentrated and the residue is chromatographed on silica gel; eluent: methylenchlorid/methanol/25% ammoniak 95:5:0.5). The pure fractions are combined, concentrated and crystallized from ethanol. 2.9 g (E)-(RS)-3-[5-(2,4-diamino-pyrimidin-5-ylmethyl)-2,3-dimethoxy-phenyl]-1-[1-(6-methylsulfanyl-pyridin-3-yl)-1H-phthalazin-2-yl]-propenone are isolated, m.p. 142-145°C. MS (ISP): 568.3 $(M+H)^+$.

The following compounds are prepared in analogy to the preparation of (E)-(RS)-3-[5-(2,4-Diamino-pyrimidin-5-ylmethyl)-2,3-dimethoxy-phenyl]-1-[1-(6-methylsulfanyl-pyridin-3-yl)-1H-phthalazin-2-yl]-propenone.

32.1 (E)-(RS)-3-[5-(2,4-Diamino-pyrimidin-5-ylmethyl)-2,3-dimethoxyphenyl]-1-[1-(4-methyl-pyridin-2-yl)-1H-phthalazin-2-yl]-propenone, m.p. 149-154°C (methanol). MS (ISP): 536.4 $(M+H)^+$.

32.2 (E)-(RS)-3-[5-(2,4-Diamino-pyrimidin-5-ylmethyl)-2,3-dimethoxyphenyl]-1-[1-(2-methyl-pyridin-3-yl)-1H-phthalazin-2-yl]-propenone, m.p. 150-155°C (acetonitrile). MS (ISP): 536.3 $(M+H)^+$.

32.3 (E)-(RS)-3-[5-(2,4-Diamino-pyrimidin-5-ylmethyl)-2,3-dimethoxyphenyl]-1-[1-(3-methoxy-phenyl)-1H-phthalazin-2-yl]-propenone, m.p. 114-118°C (methanol). MS (ISP): 551.3 $(M+H)^+$.

32.4 (E)-(RS)-3-[5-(2,4-Diamino-pyrimidin-5-ylmethyl)-2,3-dimethoxyphenyl]-1-[1-(2-methyl-pyridin-4-yl)-1H-phthalazin-2-yl]-propenone, m.p. 120°C (ethanol). MS (ISP): 536.3 $(M+H)^+$.

32.5 (E)-(RS)-3-[5-(2,4-Diamino-pyrimidin-5-ylmethyl)-2,3-dimethoxyphenyl]-1-[1-(2-methyl-pyrimidin-5-yl)-1H-phthalazin-2-yl]-propenone, m.p. 142-145°C (methanol). MS (ISP): 537.4 $(M+H)^+$.

32.6 (E)-(RS)-3-[5-(2,4-Diamino-pyrimidin-5-ylmethyl)-2,3-dimethoxyphenyl]-1-[1-(3,4,5-trimethoxy-phenyl)-1H-phthalazin-2-yl]-propenone, m.p. 190-196°C (methanol). MS (ISP): 611.3 $(M+H)^+$.

32.7 (E)-(RS)-3-[5-(2,4-Diamino-pyrimidin-5-ylmethyl)-2,3-dimethoxyphenyl]-1-[1-(6-hydroxymethyl-pyridin-3-yl)-1H-phthalazin-2-yl]-propenone, m.p. 133-136°C (methanol). MS (EI): 551 (M).

32.8 (E)-(RS)-3-[5-(2,4-Diamino-pyrimidin-5-ylmethyl)-2,3-dimethoxyphenyl]-1-(1-pyridin-4-yl-1H-phthalazin-2-yl)-propenone, m.p. 145-149°C (acetonitrile). MS (ISP): 522.2 $(M+H)^+$.

32.9 (E)-(RS)-1-[1-(6-Bromo-pyridin-3-yl)-1H-phthalazin-2-yl]-3-[5-(2,4-diamino-pyrimidin-5-ylmethyl)-2,3-dimethoxy-phenyl]-propenone, m.p. 132-138°C (methanol). MS (ISP): 602.1 $(M+H)^+$.

32.10 (E)-(RS)-3-[5-(2,4-Diamino-pyrimidin-5-ylmethyl)-2,3-dimethoxyphenyl]-1-[1-(2,4-dimethoxy-pyrimidin-5-yl)-1H-phthalazin-2-yl]-propenone, m.p. 160-165°C (methanol). MS (ISP): 583.3 $(M+H)^+$.

32.11 (E)-(RS)-3-[5-(2,4-Diamino-pyrimidin-5-ylmethyl)-2,3-dimethoxyphenyl]-1-[1-[4-(2-hydroxy-ethoxy)-phenyl]-1H-phthalazin-2-yl]-propenone, m.p. 169-172°C (methanol). MS (ISP): 581.5 $(M+H)^+$.

32.12 (E)-(RS)-3-[5-(2,4-Diamino-pyrimidin-5-ylmethyl)-2,3-dimethoxyphenyl]-1-[1-(4-methoxy-pyridin-2-yl)-1H-phthalazin-2-yl]-propenone, m.p. 125-133°C (ethanol). MS (ISP): 552.2 $(M+H)^+$.

32.13 (E)-(RS)-3-[5-(2,4-Diamino-pyrimidin-5-ylmethyl)-2,3-dimethoxyphenyl]-1-(1-[1,3]dioxolo[4,5-b]pyridin-6-yl-1H-phthalazin-2-yl)-propenone, m.p. 135-140°C (ethanol). MS (ISP): 566.2 $(M+H)^+$.

32.14 (E)-(RS)-3-[5-(2,4-Diamino-pyrimidin-5-ylmethyl)-2,3-dimethoxyphenyl]-1-[1-[6-(2-hydroxy-ethoxy)-pyridin-3-yl]-1H-phthalazin-2-yl]-propenone, m.p. 212-217°C (methanol). MS (ISP): 582.1 $(M+H)^+$.

32.15 (E)-(RS)-3-[5-(2,4-Diamino-pyrimidin-5-ylmethyl)-2,3-dimethoxyphenyl] -1-[1-[6-(2-methoxy-ethoxy)-pyridin-3-yl]-1H-phthalazin-2-yl]-propenone, m.p. 147-151°C (methanol). MS (ISP): 596.2 $(M+H)^+$.

32.16 (E)-(RS)-3-[5-(2,4-Diamino-pyrimidin-5-ylmethyl)-2,3-dimethoxyphenyl]-1-[1-[6-[2-(2-methoxy-ethoxy)-ethoxy]-pyridin-3-yl]-1H-phthalazin-2-yl]-propenone, m.p. 136-140°C (ethanol). MS (ISP): 640.4 $(M+H)^+$.

32.17 (E)-(RS)-3-[5-(2,4-Diamino-pyrimidin-5-ylmethyl)-2,3-dimethoxyphenyl]-1-[1-[6-(3-hydroxy-propoxy)-pyridin-3-yl]-1H-phthalazin-2-yl]-propenone, m.p. 173-178°C (methanol). MS (ISP): 596.2 $(M+H)^+$.

32.18 1-[1-(6-Benzyloxy-pyridin-3-yl)-1H-phthalazin-2-yl]-3-[5-(2,4-diaminopyrimidin-5-ylmethyl)-2,3-dimethoxy-phenyl]-propenone, m.p. 136-143°C (ethanol). MS (ISP): 628.2 $(M+H)^+$.

32.19 3-[5-(2,4-Diamino-pyrimidin-5-ylmethyl)-2,3-dimethoxy-phenyl]-1-{1-[6-(2-morpholin-4-yl-ethoxy)-pyridin-3-yl]-1H-phthalazin-2-yl}-propenone, m.p. 120-124°C (ethanol). MS (ISP): 651.2 $(M+H)^+$.

32.20 3-[5-(2,4-Diamino-pyrimidin-5-ylmethyl)-2,3-dimethoxy-phenyl]-1-{1-[6-(2-dimethylamino-ethoxy)-pyridin-3-yl]-1H-phthalazin-2-yl}-propenone, m.p. 110-114°C (ethanol/water). MS (ISP): 609.2 $(M+H)^+$.

32.21 (E)-(RS)-1-[1-(6-Chloro-pyridin-3-yl)-1H-phthalazin-2-yl]-3-[5-(2,4-diamino-pyrimidin-5-ylmethyl)-2,3-dimethoxy-phenyl]-propenone, m.p. 129-140°C (methanol). MS (ISP): 556.1 $(M+H)^+$.

The starting material used above are prepared as follows:

Example 33

Preparation of 5-Bromo-2-(2-methoxy-ethoxy)-pyridine

1.1 g sodium and 2.5 g 2,5-dibromo-pyridine are added to 50 ml 2-methoxy-ethanol. The reaction mixture is heated to 90°C and stirred for a further 2.5 hrs, then poured onto 75 ml of a saturated sodiumhydrogen carbonate solution and extracted three times with methylenchloride. The organic phases are combined, dried over sodium sulphate and concentrated and the residue is destilled. 2.2 g 5-Bromo-2-(2-methoxy-ethoxy)-pyridine are obtained., bp. 135°C/0.1 mbar MS (EI): 231 (M).

The following compounds are prepared in analogy to the preparation of 5-bromo-2-(2-methoxy-ethoxy)-pyridine.

33.1. 2-(5-Bromo-pyridin-2-yloxy)-ethanol, m.p. 60°C (hexane). MS (EI): 217 (M).

33.2 5-Bromo-2-[2-(2-methoxy-ethoxy)-ethoxy]-pyridine, b.p. 130°C/1 mbar MS (EI): 276 $(M+H)^+$.

33.3 3-(5-Bromo-pyridin-2-yloxy)-propan-1-ol, m.p. 58°C (hexane). MS (EI): 231 (M).

33.4 4-[2-(5-Bromo-pyridin-2-yloxy)-ethyl]-morpholine, b.p. 130°C/0.2 mbar

33.5 [2-(5-Bromo-pyridin-2-yloxy)-ethyl]-dimethyl-amine, b.p. 100°C/0.16 mbar MS (ISP): 247.1 $(M+H)^+$.

Example 34

Preparation of (E)-(RS)-5-(2-{3-[5-(2,4-Diamino-pyrimidin-5-ylmethyl)-2,3-dimethoxy-phenyl]-acryloyl}-1,2-dihydro-phthalazin-1-yl)-pyridin-2-carbonic acid amide

15.3 ml of a 1.6 M solution of butyllithium in hexane are added dropwise at -78°C within 15 min. to a solution of 5.68 g 2,5-dibromo-pyridine in 200 ml of diethylether. The reaction mixture is stirred at -78°C for 10 min. Then a solution of 2.6 g phthalazine in 100 ml tetrahydrofuran is added. The reaction mixture is stirred at -78°C for a further 10 min. Then 20.5 ml of a 1.6 M solution of butyllithium in hexane are added dropwise at -78°C within 10 min. 5 ml trimethylsilylisocyanat are added. The reaction mixture is stirred for a further 20 min. The termperature raises up to -25°C (solution A).

26.2 ml of a 1.6 M solution of butyllithium in hexane are added dropwise at -78°C within 10 min to a solution of 7.16 g (E)-3-[5-(2,4-diamino-pyrimidin-5-ylmethyl)-2,3-dimethoxy-phenyl]-acrylacid ethyl ester in 750 ml tetrahydrofuran. Solution A is added. The reaction mixture is stirred for a further 5.5 h. The termperature raises up to -18°C. 200 ml water and 300 ml of a saturated sodium chloride solution are added. The organic phases are combined, dried over sodium sulphate and concentrated and the residue is chromatographed on silica gel; eluent: methylenchlorid/methanol/25% ammoniak 95:5:0.5). The pure fractions are combined, concentrated and crystallized from ethanol. After recrystallization from ethanol 0.84 g (E)-(RS)-5-(2-{3-[5-(2,4-Diamino-pyrimidin-5-ylmethyl)-2,3-dimethoxy-phenyl]-acryloyl}-1,2-dihydro-phthalazin-1-yl)-pyridin-2-carbonsäure amide are obtained; m.p. 204-209°C (Ethanol). MS (ISP): 565.3 $(M+H)^+$.

The following compounds are prepared in analogy to the preparation of (E)-(RS)-5-(2-{3-[5-(2,4-Diamino-pyrimidin-5-ylmethyl)-2,3-dimethoxy-phenyl]-acryloyl}-1,2-dihydro-phthalazin-1-yl)-pyridin-2-carbonsäure amide

34.1 (E)-(RS)-3-[5-(2,4-Diamino-pyrimidin-5-ylmethyl)-2,3-dimethoxy-phenyl]-1-[1-(6-ethyl-pyridin-3-yl)-1H-phthalazin-2-yl]-propenone, m.p. 124-130°C (ethanol). MS (ISP): 550.2 $(M+H)^+$.

Pharmaceutical preparations can be produced in a manner known per se according to the following formulations:

Example A

| Tablets: | |
|---|---|
| Sulfamethoxazole | 400 mg |
| Compound of formula I, e.g. (E)-(RS)-3-[5-(2,4-diamino-pyrimidin-5-ylmethyl)-2,3-dimethoxy-phenyl]-1-(1-phenyl-1H-phthalazin-2-yl)-propenone | 80 mg |
| PRIMOJEL (starch derivative) | 6 mg |
| POVIDONE K30 (polyvinylpyrrolidone) | 8 mg |
| Magnesium stearate | 6 mg |
| | Total weight 500 mg |

Example B

| Tablets: | |
|---|---|
| Compound of formula I, e.g. (E)-(RS)-3-[5-(2,4-diamino-pyrimidin-5-ylmethyl)-2,3-dimethoxy-phenyl]-1-(1-furan-2-yl-1H-phthalazin-2-yl)-propenone | 100 mg |
| Corn starch | 15 mg |
| Talc | 3 mg |
| Magnesium stearate | 2 mg |
| | 120 mg |

Example C

| Injection solutions: | |
|---|---|
| Compound of formula I, e.g. E)-(RS)-3-{5-(2,4-diamino-pyrimidin-5-ylmethyl)-2,3-dimethoxy-phenyl}-1-(1-propyl-1H-phthalazin-2-yl)-propenone | 5 mg |
| Glycofurol 75 | 0.2 ml |
| Aq. bidist. sterile | ad 1.0 ml |

Example D

| Injection solutions: | |
|---|---|
| Compound of formula I, e.g. E)-(RS)-3-{5-(2,4-diamino-pyrimidin-5-ylmethyl)-2,3-dimethoxy-phenyl}-1-(1-propyl-1H-phthalazin-2-yl)-propenone | 5 mg |
| Propylene glycol | 0.5 ml |
| Aq. bidist. sterile | ad 1.0 ml |

**Claims**

**1.** Compounds of the general formula

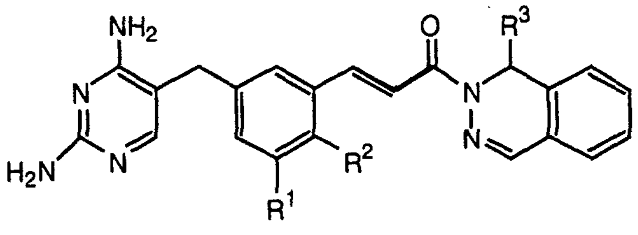

I

wherein

$R^1$ signifies $C_{1-4}$-alkoxy or $C_{1-4}$-alkoxy substituted with amino, dialkylamino, morpholino, piperidino; piperazino, hydroxy, halide, nitrile, thiocyanato, sulfato, methylsulfanyl, oxo, carboxy, carbamino, carbalkoxy groups, alkoxy, morpholinoalkoxy or piperidinoalkoxy;

$R^2$ signifies hydroxy, $C_{1-4}$-alkoxy or $C_{1-4}$-alkoxy substituted with amino, dialkylamino, morpholino, piperidino, piperazino, hydroxy, halide, nitrile, thiocyanato, sulfato, methylsulfanyl, oxo, carboxy, carbamino, carbalkoxy groups, alkoxy, morpholinoalkoxy or piperidinoalkoxy;

$R^3$ signifies hydrogen, cyano, $C_{1-6}$-alkyl, substituted $C_{1-6}$-alkyl, up to $C_6$-alkenyl, substituted up to $C_6$-

alkenyl, $C_{3-6}$-cycloalkyl, substituted $C_{3-6}$-cycloalkyl, bicyclyl, aryl, substituted aryl, heterocyclyl, substituted heterocyclyl, heteroaryl, substituted heteroaryl, aralkyl, substituted aralkyl, aryl-Q-alkyl, substituted aryl-Q-alkyl, heterocyclylalkyl, substituted heterocyclylalkyl or a group of the formula-$CR^4R^{4'}COR^5$,
wherein substituted $C_{1-6}$-alkyl or substituted $C_{3-6}$-cycloalkyl are substituted with amino, dialkylamino, morpholino, piperidino, piperazino, hydroxy, halide, nitrile, thiocyanato, sulfato, methylsulfanyl, oxo, carboxy, carbamino, carbalkoxy groups, alkoxy, morpholinoalkoxy or piperidinoalkoxy, and
wherein substituted up to $C_6$-alkenyl is subsituted with cyano or acryloyl, and
wherein heterocyclyl signifies 4 to 6 membered rings with 1 to 3 N, O and/or S atoms, and
wherein substituted aryl, substituted heterocyclyl or substituted heteroaryl are substituted with phenyl, $C_{1-6}$-alkyl, substituted $C_{1-6}$-alkyl, $C_{3-6}$ cycloalkyl, hydroxy, cyano, thiocyanato, amino, hydroxyalkyl (optionally esterified with amino acids or sulphuric acid), halogen, $C_{1-4}$-alkoxy, $C_{1-4}$-alkoxycarbonyl, di($C_{1-6}$-alkyl)amino, carbamoyl, mono- or di-$C_{1-6}$-alkylcarbamoyl, $C_{1-6}$-alkylsulphanoyl, $C_{1-6}$-alkylsulphonyl, sulphamoyl, N-mono- or di-$C_{1-6}$-alkylsulphamoyl, heterocyclyl, or heterocyclyl -$C_{1-6}$-alkyl, and
wherein aryl signifies b-membered mono- or poly-nucleus aromatic groups
wherein heteroaryl signifies 5- or 6- membered, mono- or poly-nuclear heteroaromatic groups with preferably 5-13 carbon atoms and 1-4 hetero atoms;
wherein bicyclyl denotes adamantlyl or bicyclo[2.2.1]hept-2endo and/or 2exo-yl

Q signifies -SO- or $SO_2$;
$R^4$, $R^{4'}$ each independently signify hydrogen, alkyl, aryl, substituted aryl, heterocyclyl or substituted heterocyclyl,
wherein heterocyclyl, substituted aryl and substituted heterocylyl are as defined for $R^3$;
$R^5$ signifies hydrogen, alkyl, alkoxy, aryl, substituted aryl, heterocyclyl or substituted heterocyclyl,
wherein heterocyclyl, substituted aryl, and substituted heterocylyl are as defined for $R^3$;
$R^4$ and $R^5$ together can form a group $-(CH_2)_n-$, and
n is a whole number of 2-5,

as well as pharmaceutically usable salts thereof.

**2.** Compounds according to claim 1, **characterized in that** $R^1$ and $R^2$ signify $C_{1-4}$-alkoxy or substituted $C_{1-4}$-alkoxy,
wherein substituted $C_{1-4}$-alkoxy is as defined in claim 1.

**3.** Compounds according to claim 1 or 2, **characterized in that** $R^3$ is $C_{1-6}$-alkyl or substituted $C_{1-6}$-alkyl,
wherein substituted $C_{1-6}$-alkyl is as defined in claim 1.

**4.** Compounds according to claim 1 or 2, **characterized in that** $R^3$ signifies phenyl or substituted phenyl,
wherein substituted phenyl is substituted with phenyl, $C_{1-6}$-alkyl, substituted $C_{1-6}$-alkyl, $C_{3-6}$ cycloalkyl, hydroxy, cyano, thiocyanato, amino, hydroxyalkyl (optionally esterified with amino acids or sulphuric acid), halogen, $C_{1-4}$-alkoxy, $C_{1-4}$-alkoxycarbonyl, di($C_{1-6}$-alkyl)amino, carbamoyl, mono- or di- $C_{1-6}$-alkylcarbamoyl, $C_{1-6}$-alkylsulphanoyl, $C_{1-6}$-alkylsulphonyl, sulphamoyl, N-mono- or di-$C_{1-6}$-alkylsulphamoyl, heterocyclyl, or heterocyclyl-$C_{1-6}$-alkyl.

**5.** Compounds according to claim 1 or 2, **characterized in that** $R^3$ signifies furyl, pyridyl, substituted pyridyl, pyrimidinyl or substituted pyrimidinyl,
wherein substituted pyridyl or substituted pyrimidinyl are substituted with phenyl, $C_{1-6}$-alkyl, substituted $C_{1-6}$-alkyl, $C_{3-6}$ cycloalkyl, hydroxy, cyano, thiocyanato, amino, hydroxyalkyl (optionally esterified with amino acids or sulphuric acid), halogen, $C_{1-4}$-alkoxy, $C_{1-4}$-alkoxycarbonyl, di($C_{1-6}$-alkyl)amino, carbamoyl, mono- or di- $C_{1-6}$-alkylcarbamoyl, $C_{1-6}$-alkylsulphanoyl, $C_{1-6}$-alkylsulphonyl, sulphamoyl, N-mono- or di-$C_{1-6}$-alkylsulphamoyl, heterocyclyl, or heterocyclyl-$C_{1-6}$-alkyl.

**6.** The compounds as claimed in claim 1, which compounds are selected from:

(E)-(RS)-3- [5-(2,4-diamino-pyrimidin-5-ylmethyl)-2,3-dimethoxy-phenyl]-1-(1-phenyl-1H-phthalazin-2-yl)-propenone,
(E)-(RS)-3-[5-(2,4-diamino-pyrimidin-5-ylmethyl)-2,3-dimethoxy-phenyl]-1-(1-furan-2-yl-1H-phthalazin-2-yl)-propenone,
(E)-(RS)-3-[5-(2,4-diamino-pyrimidin-5-ylmethyl)-2,3-dimethoxy-phenyl]-1-[1-(4-hydroxymethyl-phenyl)- 1H-phthalazin-2-yl]-propenone,
(E)-(RS)-3-[5-(2,4-diamino-pyrimidin-5-ylmethyl)-2,3-dimethoxy-phenyl]-1-[1-(4-hydroxy-phenyl)-1H-phthalazin-2-yl]-propenone,
(E)-(RS)-3-[5-(2,4-diamino-pyrimidin-5-ylmethyl)-2,3-dimethoxy-phenyl]-1-(1-ethyl-1H-phthalazin-2-yl)-propenone,
(E)-(RS)-3-[5-(2,4-diamino-pyrimidin-5-ylmethyl)-2,3-dimethoxy-phenyl]-1-[1-(4-hydroxy-butyl)-1H-phthalazin-2-yl] -propenone,
(E)-(R)-3-[5-(2,4-Diamino-pyrimidin-5-ylmethyl)-2,3-dimethoxy-phenyl]-1-[1-(6-methyl-pyridin-3-yl)-1H-phthalazin-2-yl] -propenone,
(E)-(RS)-3-[5-(2,4-Diamino-pyrimidin-5-ylmethyl)-2,3-dimethoxy-phenyl]-1-[1-(6-methyl-pyridin-3-yl)-1H-phthalazin-2-yl]-propenone,
(E)-(RS)-3-[5-(2,4-Diamino-pyrimidin-5-ylmethyl)-2,3-dimethoxy-phenyl]-1-[1-(6-ethyl-pyridin-3-yl)-1H-phthalazin-2-yl]-propenone,
(E)-(RS)-3-[5-(2,4-Diamino-pyrimidin-5-ylmethyl)-2,3-dimethoxy-phenyl]-1-[1-(2-methyl-pyrimidin-5-yl)-1H-phthalazin-2-yl]-propenone,

as well as pharmaceutically usable salts of these compounds.

**7.** Compounds according to any one of claims 1-6 for use as medicaments.

**8.** A process for the manufacture of the compounds in accordance with any one of claims 1-6, **characterized by**

(a) reacting a compound of the general formula

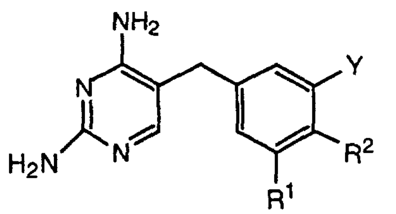

II

wherein $R^1$ and $R^2$ are as defined in claim 1 and Y signifies a leaving group, with a compound of the general formula

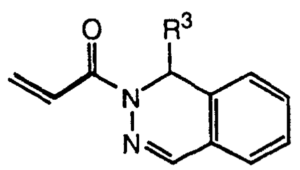

III

wherein $R^3$ is as defined in claim 1, or
(b) reacting a phthalazine with a silyl enol ether of formula V or an enamine of formula VI in the presence of a reactive acrylic acid derivative:

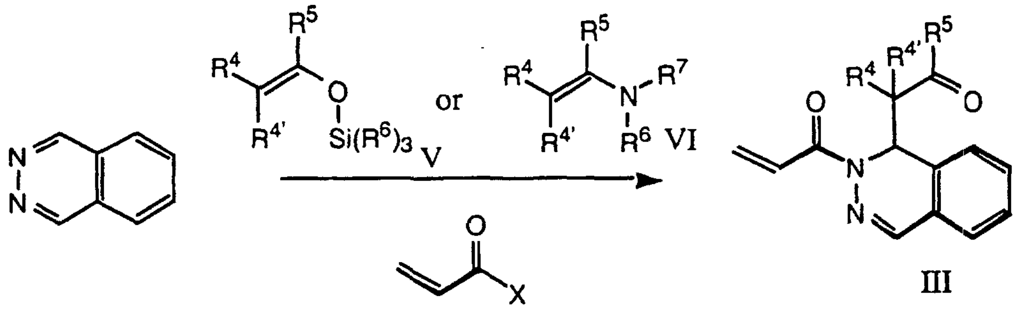


V

VI

III

wherein

| | |
|---|---|
| $R^4$, $R^{4'}$, $R^5$ and n | are as defined in claim 1, |
| $R^6$ and $R^7$ | signify $C_{1-6}$-alkyl or substituted $C_{1-6}$-alkyl, wherein substituted $C_{1-6}$-alkyl is as defined in claim 1; and |
| $R^6$ and $R^7$ | in the case of enamines of formula VI can also together form a ring |
| X | signifies a halogen atom, preferably chlorine or bromine. |

**9.** A medicament containing a compound according to any one of claims 1-6 and a therapeutically inert carrier.

**10.** A pharmaceutical preparation containing a compound of formula I and a sulphonamide as well as usual pharmaceutical carrier materials.

**11.** A preparation according to claim 10 containing a compound of formula I and a sulphonamide in the ratio of preferred ratio being 1:10 to 1:2 parts by weight.

**12.** Use of compounds of formula I and a sulphonamide for the production of pharmaceutical preparations for the treatment of infections.

**13.** Use of compounds according to any one of claims 1-6 as medicaments, especially in infectious diseases.

**14.** Use of compounds according any one of claims 1-6 in the production of antibiotically-active medicaments.

**Patentansprüche**

**1.** Verbindungen der allgemeinen Formel

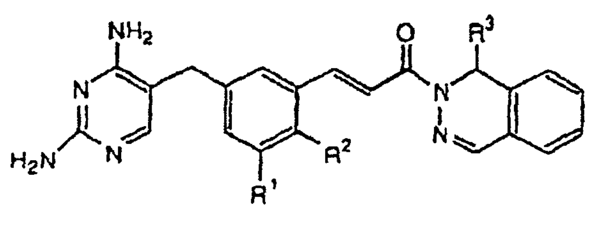

I

worin

$R^1$ eine $C_1$-$C_4$-Alkoxygruppe oder eine mit Amino, Dialkylamino, Morpholino, Piperidino, Piperazino, Hydroxy, Halogenid, Nitril, Thiocyanato, Sulfato, Methylsulfanyl, Oxo, Carboxy, Carbamino, Carbalkoxy, Alkoxy, Morpholinoalkoxy oder Piperidinoalkoxy substituierte $C_1$-$C_4$-Alkoxygruppe bedeutet;
$R^2$ eine Hydroxy-, $C_1$-$C_4$-Alkoxy- oder mit Amino, Dialkylamino, Morpholino, Piperidino, Piperazino, Hydroxy, Halogenid, Nitril, Thiocyanato, Sulfato, Methylsulfanyl, Oxo, Carboxy, Carbamino, Carbalkoxy, Alkoxy, Mor-

pholinoalkoxy oder Piperidinoalkoxy substituierte $C_1$-$C_4$-Alkoxygruppe bedeutet;
$R^3$ Wasserstoff, eine Cyano-, $C_1$-$C_6$-Alkyl-, substituierte $C_1$-$C_6$-Alkyl-, bis zu $C_6$-Alkenyl-, substituierte bis zu $C_6$-Alkenyl-, $C_3$-$C_6$-Cycloalkyl-, substituierte $C_3$-$C_6$-Cycloalkyl-, Bicyclyl-, Aryl-, substituierte Aryl-, Heterocyclyl-, substituierte Heterocyclyl-, Heteroaryl-, substituierte Heteroaryl-, Aralkyl-, substituierte Aralkyl-, Aryl-Q-alkyl-, substituierte Aryl-Q-alkyl-, Heterocyclylalkyl-, substituierte Heterocyclylalkylgruppe oder eine Gruppe der Formel $-CR^4R^{4'}COR^5$ bedeutet,

wobei die substituierten $C_1$-$C_6$-Alkyl- oder substituierten $C_3$-$C_6$-Cycloalkylgruppen mit Amino, Dialkylamino, Morpholino, Piperidino, Piperazino, Hydroxy, Halogenid, Nitril, Thiocyanato, Sulfato, Methylsulfanyl, Oxo, Carboxy, Carbamino, Carbalkoxy, Alkoxy, Morpholinoalkoxy oder Piperidinoalkoxy substituiert sind und
wobei die substituierte bis zu $C_6$-Alkenylgruppe mit Cyano oder Acryloyl substituiert ist und
wobei eine Heterocyclylgruppe 4- bis 6-gliedrige Ringe mit 1 bis 3 N-, O- und/oder S-Atomen bedeutet und
wobei substituierte Aryl-, substituierte Heterocyclyloder substituierte Heteroarylgruppen mit Phenyl, $C_1$-$C_6$-Alkyl, substituierten $C_1$-$C_6$-Alkyl, $C_3$-$C_6$-Cycloalkyl, Hydroxy, Cyano, Thiocyanato, Amino, Hydroxyalkyl (gegebenenfalls verestert mit Aminosäuren oder Schwefelsäure), Halogen, $C_1$-$C_4$-Alkoxy, $C_1$-$C_4$-Alkoxycarbonyl, Di($C_1$-$C_6$)alkylamino, Carbamoyl, Monooder Di($C_1$-$C_6$)alkylcarbamoyl, $C_1$-$C_6$-Alkylsulfanoyl, $C_1$-$C_6$-Alkylsulfonyl, Sulfamoyl, N-Mono- oder Di-$C_1$-$C_6$alkylsulfamoyl, Heterocyclyl oder Heterocyclyl-$C_1$-$C_6$alkyl substituiert sind und
wobei eine Arylgruppe 6-gliedrige ein- oder mehrkernige aromatische Gruppen bedeutet,
wobei eine Heteroarylgruppe 5- oder 6-gliedrige einoder mehrkernige heteroaromatische Gruppen mit bevorzugt 5 bis 13 Kohlenstoffatomen und 1 bis 4 Heteroatomen bedeutet,
wobei eine Bicyclylgruppe eine Adamantyl- oder Bicyclo[2.2.1]hept-2-endo- und/oder -2-exo-yl-Gruppe bedeutet;
Q -SO- oder $SO_2$ bedeutet;

$R^4$, $R^{4'}$ jeweils unabhängig Wasserstoff, Alkyl-, Aryl-, substituierte Aryl-, Heterocyclyl- oder substituierte Heterocyclylgruppen bedeuten,
wobei Heterocyclyl-, substituierte Aryl- und substituierte Heterocyclylgruppen wie für $R^3$ definiert sind;
$R^5$ Wasserstoff, eine Alkyl-, Alkoxy-, Aryl-, substituierte Aryl-, Heterocyclyl- oder substituierte Heterocyclylgruppe bedeutet,
wobei Heterocyclyl-, substituierte Aryl- und substituierte Heterocyclylgruppen wie für $R^3$ definiert sind;
$R^4$ und $R^5$ zusammen eine Gruppe $-(CH_2)_n-$ bilden können und
n eine ganze Zahl von 2 bis 5 ist ebenso wie pharmazeutisch nützliche Salze davon.

2. Verbindungen nach Anspruch 1, **dadurch gekennzeichnet, dass** $R^1$ und $R^2$ $C_1$-$C_4$-Alkoxygruppen oder substituierte $C_1$-$C_4$-Alkoxygruppen bedeuten, wobei substituierte $C_1$-$C_4$-Alkoxygruppen wie in Anspruch 1 definiert sind.

3. Verbindungen nach Anspruch 1 oder Anspruch 2, **dadurch gekennzeichnet, dass** $R^3$ eine $C_1$-$C_6$-Alkyl- oder substituierte $C_1$-$C_6$-Alkylgruppe ist, wobei eine substituierte $C_1$-$C_6$-Alkylgruppe wie in Anspruch 1 definiert ist.

4. Verbindungen nach Anspruch 1 oder Anspruch 2, **dadurch gekennzeichnet, dass** $R^3$ eine Phenyl- oder substituierte Phenylgruppe bedeutet, wobei eine substituierte Phenylgruppe mit Phenyl-, $C_1$-$C_6$-Alkyl-, substituierten $C_1$-$C_6$-Alkyl-, $C_3$-$C_6$-Cycloalkyl-, Hydroxy-, Cyano-, Thiocyanato-, Amino-, Hydroxyalkyl- (gegebenenfalls verestert mit Aminosäuren oder Schwefelsäure), Halogen-, $C_1$-$C_4$-Alkoxy-, $C_1$-$C_4$-Alkoxycarbonyl-, Di($C_1$-$C_6$alkyl)amino-, Carbamoyl-, Mono- oder Di-$C_1$-$C_6$alkylcarbamoyl-, $C_1$-$C_6$-Alkylsulfanoyl-, $C_1$-$C_6$-Alkylsulfonyl-, Sulfamoyl-, N-Mono- oder Di-$C_1$-$C_6$alkylsulfamoyl-, Heterocyclyl- oder Heterocyclyl-$C_1$-$C_6$alkylresten substituiert ist.

5. Verbindungen nach Anspruch 1 oder Anspruch 2, **dadurch gekennzeichnet, dass** $R^3$ eine Furyl-, Pyridyl-, substituierte Pyridyl-, Pyrimidinyl- oder substituierte Pyrimidinylgruppe bedeutet, wobei eine substituierte Pyridyl- oder substituierte Pyrimidinylgruppe mit Phenyl-, $C_1$-$C_6$-Alkyl-, substituierten $C_1$-$C_6$-Alkyl-, $C_3$-$C_6$-Cycloalkyl-, Hydroxy-, Cyano-, Thiocyanato-, Amino-, Hydroxyalkyl- (gegebenenfalls verestert mit Aminosäuren oder Schwefelsäure), Halogen-, $C_1$-$C_4$-Alkoxy-, $C_1$-$C_4$-Alkoxycarbonyl-, Di($C_1$-$C_6$-alkyl)amino-, Carbamoyl-, Mono- oder Di-$C_1$-$C_6$-alkylcarbamoyl-, $C_1$-$C_6$-Alkylsulfanoyl-, $C_1$-$C_6$-Alkylsulfonyl-, Sulfamoyl-, N-Mono- oder Di-$C_1$-$C_6$-alkylsulfamoyl-, Heterocyclyl- oder Heterocyclyl-$C_1$-$C_6$-alkylresten substituiert ist.

6. Verbindungen nach Anspruch 1, wobei die Verbindungen ausgewählt sind aus:

(E)-(RS)-3-[5-(2,4-Diaminopyrimidin-5-ylmethyl)-2,3-dimethoxyphenyl]-1-(1-phenyl-1H-phthalazin-2-yl)propenon,
(E)-(RS)-3-[5-(2,4-Diaminopyrimidin-5-ylmethyl)-2,3-dimethoxyphenyl]-1-(1-furan-2-yl-1H-phthalazin-2-yl)

propenon,
(E)-(RS)-3-[5-(2,4-Diaminopyrimidin-5-ylmethyl)-2,3-dimethoxyphenyl]-1-[1-(4-hydroxymethylphenyl)-1H-phthalazin-2-yl]propenon,
(E)-(RS)-3-[5-(2,4-Diaminopyrimidin-5-ylmethyl)-2,3-dimethoxyphenyl]-1-[1-(4-hydroxyphenyl)-1H-phthalazin-2-yl]propenon,
(E)-(RS)-3-[5-(2,4-Diaminopyrimidin-5-ylmethyl)-2,3-dimethoxyphenyl]-1-(1-ethyl-1H-phthalazin-2-yl)propenon,
(E)-(RS)-3-[5-(2, 4-Diaminopyrimidin-5-ylmethyl)-2,3-dimethoxyphenyl]-1-[1-(4-hydroxybutyl)-1H-phthalazin-2-yl]propenon,
(E)-(R)-3-[5-(2,4-Diaminopyrimidin-5-ylmethyl)-2,3-dimethoxyphenyl]-1-[1-(6-methylpyridin-3-yl)-1H-phthalazin-2-yl]propenon,
(E)-(RS)-3-[5-(2,4-Diaminopyrimidin-5-ylmethyl)-2,3-dimethoxyphenyl]-1-[1-(6-methylpyridin-3-yl)-1H-phthalazin-2-yl]propenon,
(E)-(RS)-3-[5-(2,4-Diaminopyrimidin-5-ylmethyl)-2,3-dimethoxyphenyl]-1-[1-(6-ethylpyridin-3-yl)-1H-phthalazin-2-yl]propenon,
(E)-(RS)-3-[5-(2,4-Diaminopyrimidin-5-ylmethyl)-2,3-dimethoxyphenyl]-1-[1-(2-methylpyrimidin-5-yl)-1H-phthalazin-2-yl]propenon,

ebenso wie die pharmazeutisch annehmbaren Salze dieser Verbindungen.

**7.** Verbindungen nach einem der Ansprüche 1 bis 6 zur Verwendung als Arzneimittel.

**8.** Verfahren zur Herstellung der Verbindungen gemäß einem der Ansprüche 1 bis 6, **dadurch gekennzeichnet, dass**

(a) eine Verbindung der allgemeinen Formel

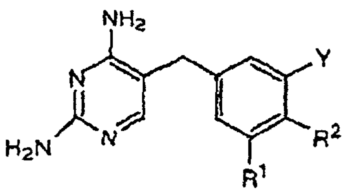

II

wobei $R^1$ und $R^2$ wie in Anspruch 1 definiert sind und Y eine Abgangsgruppe bedeutet, mit einer Verbindung der allgemeinen Formel

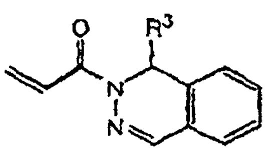

III

worin $R^3$ wie in Anspruch 1 definiert ist, umgesetzt wird, oder
(b) ein Phthalazin mit einem Silylenolether der Formel V oder einem Enamin der Formel VI in Gegenwart eines reaktiven Acrylsäurederivats

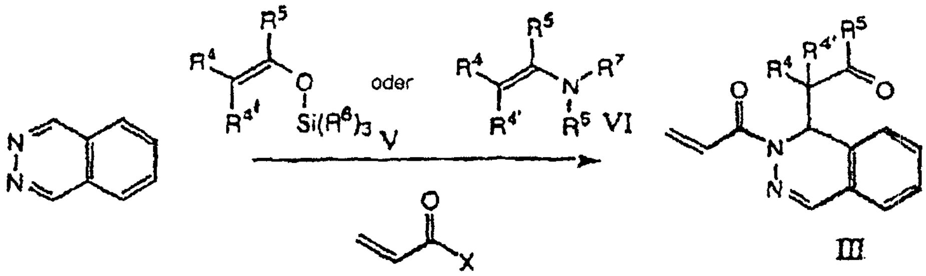


III

umgesetzt wird,

wobei $R^4$, $R^{4'}$, $R^5$ und n wie in Anspruch 1 definiert sind, $R^6$ und $R^7$ $C_1$-$C_6$-Alkyl- oder substituierte $C_1$-$C_6$-Alkylreste bedeuten, worin ein substituierter $C_1$-$C_6$-Alkylrest wie in Anspruch 1 definiert ist; und $R^6$ und $R^7$ in dem Fall der Enamine der Formel VI auch zusammen einen Ring bilden können, X ein Halogenatom, bevorzugt Chlor oder Brom bedeutet.

**9.** Arzneimittel enthaltend eine Verbindung nach einem der Ansprüche 1 bis 6 und einen therapeutisch inerten Träger.

**10.** Pharmazeutisches Präparat enthaltend eine Verbindung der Formel I und ein Sulfonamid ebenso wie übliche pharmazeutische Trägermaterialien.

**11.** Präparat nach Anspruch 10 enthaltend eine Verbindung der Formel I und ein Sulfonamid in einem bevorzugten Verhältnis von 1:10 bis 1:2 Teile pro Gewicht.

**12.** Verwendung der Verbindungen der Formel I und eines Sulfonamids zur Herstellung von pharmazeutischen Präparaten zur Behandlung von Infektionen.

**13.** Verwendung von Verbindungen nach einem der Ansprüche 1 bis 6 als Arzneimittel, insbesondere für Infektionskrankheiten.

**14.** Verwendung von Verbindungen nach einem der Ansprüche 1 bis 6 zur Herstellung von antibiotisch aktiven Medikamenten.

**Revendications**

**1.** Composés de formule générale

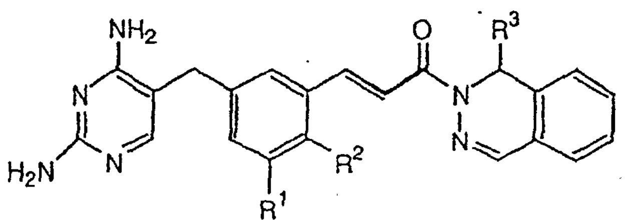

I

dans laquelle :

$R^1$ signifie alcoxy en C1-4 ou alcoxy en C1-4 substitué par des groupes amino, dialkylamino, morpholino, pipéridino; pipérazino, hydroxy, halogénure, nitrile, thiocyanato, sulfato, méthylsulfanyle, oxo, carboxy, carbamino, carbalcoxy, alcoxy, morpholinoalcoxy ou pipéridinoalcoxy ;

$R^2$ signifie hydroxy, alcoxy en C1-4 ou alcoxy en C1-4 substitué par des groupes amino, dialkylamino, morpholino, pipéridino ; pipérazino, hydroxy, halogénure, nitrile, thiocyanato, sulfato, méthylsulfanyle, oxo, carboxy, carbamino, carbalcoxy, alcoxy, morpholinoalcoxy ou pipéridinoalcoxy ;

$R^3$ signifie l'hydrogène, cyano, alkyle en C1-6, alkyle en C1-6 substitué, alcényle jusqu'en C6, alcényle jusqu'en C6 substitué, cycloalkyle en C3-6, cycloalkyle substitué en C3-6, bicyclyle, aryle, aryle substitué, hétérocyclyle, hétérocyclyle substitué, hétéroaryle, hétéroaryle substitué, aralkyle, aralkyle substitué, aryl-Q-alkyle, aryl-Q-alkyle substitué, hétérocycloalkyle, hétérocycloalkyle substitué ou un groupe de formule $-CR^4R^{4'}COR^5$,

où les groupes alkyle en C1-6 substitué ou cycloalkyle en C3-6 substitué sont substitués par des groupes amino, dialkylamino, morpholino, pipéridino; pipérazino, hydroxy, halogénure, nitrile, thiocyanato, sulfato, méthylsulfanyle, oxo, carboxy, carbamino, carbalcoxy, alcoxy, morpholinoalcoxy ou pipéridinoalcoxy et où le groupe alcényle jusqu'en C6 substitué est substitué par un groupe cyano ou acryloyle, et
où le groupe hétérocyclyle signifie des noyaux à 4-6 éléments avec 1 à 3 atomes N, O et/ou S, et
où les groupes aryle substitué, hétérocyclyle substitué ou hétéroaryle substitué sont substitués par les phényle, alkyle en C1-6, alkyle substitué en C1-6, cycloalkyle en C3-6, hydroxy, cyano, thiocyanato, amino, hydroxyalkyle (facultativement estérifié par des acides aminés ou l'acide sulfurique), halogène, alcoxy en C1-4, alcoxycarbonyle en C1-4, dialkylamino en C1-6, carbamoyle, mono ou dialkylcarbamoyle en C1-6, alkylsulfanoyle en C1-6, alkylsulfonyle en C1-6, sulfamoyle, N-mono ou di-alkylsulfamoyle en C1-6, hétérocyclyle ou hétérocyclyl-alkyle en C1-6, et où aryle signifie des groupes aromatiques mono ou poly nucléaires à 6 éléments,
où le groupe hétéroaryle signifie des groupes hétéroaromatiques mono ou poly nucléaires à 5 ou 6 éléments avec de préférence 5 à 13 atomes de carbone et 1 à 4 hétéro-atomes; où le groupe bicyclyle signifie un adamantyle ou un bicyclo[2.2.1]hept-2-endo et/ou 2-exo-yle

Q signifie $-SO$ ou $-SO_2$ ;

$R^4$, $R^{4'}$ signifient chacun indépendamment l'hydrogène, alkyle, aryle, aryle substitué, hétérocyclyle ou hétérocyclyle substitué,
où les groupes hétérocyclyle, aryle substitué et hétérocyclyle substitué sont comme défini pour $R^3$ ;

$R^5$ signifie l'hydrogène, alkyle, alcoxy, aryle, aryle substitué, hétérocyclyle ou hétérocyclyle substitué,
où le groupe hétérocyclyle, aryle substitué et hétérocyclyle substitué est comme défini pour $R^3$ ;

$R^4$ et $R^5$ ensemble peuvent former un groupe $(CH_2)_n$- et

n est un nombre entier de 2 à 5,

ainsi que leurs sels pharmaceutiquement acceptables.

2. Composés selon la revendication 1, **caractérisés en ce que** $R^1$ et $R^2$ signifient alcoxy en C1-4 ou alcoxy en C1-4 substitué,
où le groupe alcoxy en C1-4 substitué est tel que défini dans la revendication 1.

3. Composés selon la revendication 1 ou 2, **caractérisés en ce que** $R^3$ est un alkyle en C1-6 ou un alkyle en C1-6 substitué,
où le groupe alkyle substitué en C1-6 est tel que défini dans la revendication 1.

4. Composés selon la revendication 1 ou 2, **caractérisés en ce que** $R^3$ signifie phényle ou phényle substitué,
où le groupe le phényle substitué est substitué par un groupe phényle, alkyle en C1-6, alkyle en C1-6 substitué, cycloalkyle en C3-6, hydroxy, cyano, thiocyanato, amino, hydroxyalkyle (facultativement estérifié par des acides aminés ou l'acide sulfurique), halogène, alcoxy en C1-4, alcoxycarbonyle en C1-4, dialkylamino en C1-6, carbamoyle, mono ou dialkylcarbamoyle en C1-6, alkylsulfanoyle en C1-6, alkylsulfonyle en C1-6, sulfamoyle, N-mono ou di-alkylsulfamoyle en C1-6, hétérocyclyle ou hétérocyclyl-alkyle en C1-6.

5. Composés selon la revendication 1 ou 2, **caractérisés en ce que** $R^3$ signifie un groupe furyle, pyridyle, pyridyle substitué, pyrimidinyle ou pyrimydinyle substitué,
où le groupe pyridyle substitué ou pyrimidinyle substitué est substitué par les phényle, alkyle en C1-6, alkyle en C1-6 substitué, cycloalkyle en C3-6, hydroxy, cyano, thiocyanato, amino, hydroxyalkyle (facultativement estérifié par des acides aminés ou l'acide sulfurique), halogène, alcoxy en C1-4, alcoxycarbonyle en C1-4, dialkylamino

en C1-6, carbamoyle, mono ou dialkylcarbamoyle en C1-6, alkylsulfanoyle en C1-6, alkylsulfonyle en C1-6, sulfamoyle, N-mono ou di-alkylsulfamoyle en C1-6, hétérocyclyle ou hétérocyclyl-alkyle en C1-6.

**6.** Composés selon la revendication 1, ces composés étant choisis parmi les

(E)-(RS)-3-[5-(2,4-diamino-pyrimidin-5-ylméthyl)-2,3-diméthoxyphényl]-1-(1- phényl-1H-phtalazin-2-yl)-propénone,
(E)-(RS)-3-[5-(2,4-diamino-pyrimidin-5-ylméthyl)-2,3-diméthoxyphényl]-1-(1- furan-2-yl-1H-phtalazin-2-yl)-propénone,
(E)-(RS)-3-[5-(2,4-diamino-pyrimidin-5-ylméthyl)-2,3-diméthoxyphenyl]-1-[1-(4-hydroxyméthyl-phényl)-1H-phalazin-2-yl]-propénone,
(E)-(RS)-3-[5-(2,4-diamino-pyrimidin-5-ylméthyl)-2,3-diméthoxyphényl]-1-[1- (4-hydroxy-phényl)-1H-phtalazin-2-yl]-propénone,
(E)-(RS)-3-[5-(2,4-diamino-pyrimidin-5-ylméthyl)-2,3-diméthoxyphényl]-1-(1- éthyl-1H-phtalazin-2-yl)-propénone,
(E)-(RS)-3-[5-(2,4-diamino-pyrimidin-5-ylméthyl)-2,3-diméthoxyphényl]-1-[1- (4-hydroxy-butyl)-1H-phtalazin-2-yl]-propénone,
(E)-(R)-3-[5-(2,4-Diamino-pyrimidin-5-ylméthyl)-2,3-diméthoxyphényl]-1-[1-(6-méthyl-pyridin-3-yl)-1H-phtalazin-2-yl]-propénone,
(E)-(RS)-3-[5-(2,4-Diamino-pyrimidin-5-ylméthyl)-2,3-diméthoxy phényl]-1-[1-(6-méthyl-pyridin-3-yl)-1H-phtalazin-2-yl]-propénone,
(E)-(RS)-3-[5-(2,4-Diamino-pyrimidin-5-ylméthyl)-2,3-diméthoxyphényl]-1-[1-(6-éthyl-pyridin-3-yl)-1H-phtalazin-2-yl]-propénone,
(E)-(RS)-3-[5-(2,4-Diamino-pyrimidin-5-ylméthyl)-2,3-diméthoxyphényl]-1-[1-(2-méthyl-pyrimidin-5-yl)-1H-phtalazin-2-yl]-propénone,

ainsi que leurs sels pharmaceutiquement acceptables.

**7.** Composés selon l'une quelconque des revendications 1 à 6 pour une utilisation comme médicaments.

**8.** Procédé pour la fabrication des composés selon l'une quelconque des revendications 1 à 6, **caractérisé par**

(a) la réaction d'un composé de formule générale

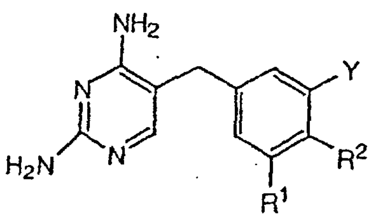

II

dans laquelle $R^1$ et $R^2$ sont comme défini dans la revendication 1 et Y signifie un groupe partant, avec un composé de formule générale :

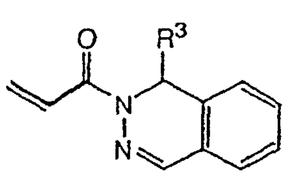

III

dans laquelle $R^3$ est comme défini dans la revendication 1, ou

(b) la réaction d'une phtalazine avec un éther de silylénol de formule V

ou une énamine de formule VI en présence d'un dérivé d'acide acrylique réactif :

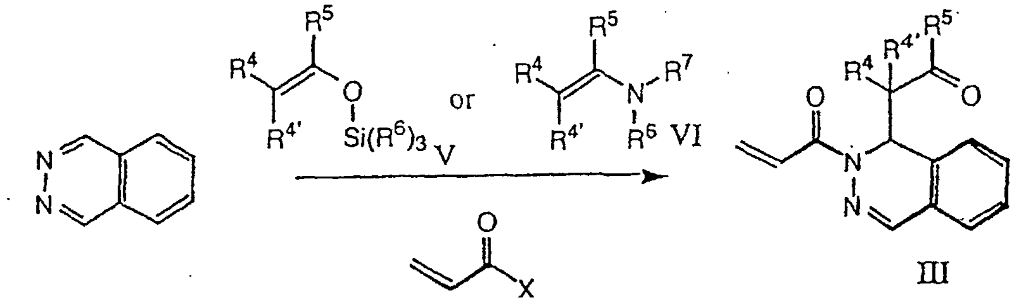

dans laquelle

$R^4$, $R^{4'}$, $R^5$ et n sont comme défini dans la revendication 1,

$R^6$ et $R^7$ signifient un groupe alkyle en C1-6 ou alkyle en C1-6 substitué, où le groupe alkyle en C1-6 substitué est tel que défini dans la revendication 1 ; et

$R^6$ et $R^7$ dans le cas des énamines de formule VI peuvent également former ensemble un noyau,

X signifie un atome d'halogène, de préférence le chlore ou le brome.

**9.** Médicament contenant un composé selon l'une quelconque des revendications 1 à 6, et un véhicule inerte sur le plan thérapeutique.

**10.** Préparation pharmaceutique contenant un composé de formule I et un sulfonamide ainsi que des véhicules pharmaceutiquement acceptables.

**11.** Préparation selon la revendication 10 contenant un composé de formule I et un sulfonamide en un rapport de rapport préféré de 1: 10 à 1: 2 parties en poids.

**12.** Utilisation de composés de formule I et d'un sulfonamide pour la fabrication de préparations pharmaceutiques destinées au traitement des infections.

**13.** Utilisation de composés selon l'une quelconque des revendications 1 à 6, comme médicaments, en particulier pour les maladies infectieuses.

**14.** Utilisation de composés selon l'une quelconque des revendications 1 à 6, pour la fabrication de médicaments actifs sur le plan antibiotique.